# FASCICULE DE BREVET EUROPEEN

(11) **EP 0 719 775 B1**
(45) Date de publication et mention de la délivrance du brevet: **12.07.2000**
(21) Numéro de dépôt: 95402969.0
(22) Date de dépôt: 28.12.1995
(51) Int. Cl.: C07D 417/12, A61K 31/445

(54) **Dérivés de Phényl-4-thiazoles substitués, procédé pour leur préparation et compositions pharmaceutiques les contenant**
Substituierte 4-Phenyl-Thiazole Derivate, Verfahren zu deren Herstellung und pharmazeutische Zusammenstellungen die sie enthalten
Substituted 4-phenyl thiazole derivatives, process for their preparation and pharmaceutical compositions containing them

(30) Priorité: 28.12.1994 FR 9415805
(43) Date de publication de la demande: 03.07.1996
(73) Titulaire: SANOFI-SYNTHELABO, 75635 Paris Cédex 13 (FR)
(72) Inventeur: Badorc, Alain, F-31120 Roquettes (FR); Bordes, Marie-Françoise, F-31860 Labarthe sur Leze (FR); De Cointet, Paul, F-31400 Toulouse (FR); Herbert, Jean-Marc, F-31170 Tournefeuille (FR); Maffrand, Jean-Pierre, F-31120 Portet/Garonne (FR)
(74) Mandataire: Le Guen, Gérard

(56) Documents cités:
- EP-A- 0 542 363
- WO-A-91/09857

## Description

La présente invention concerne des nouveaux dérivés du 2-amino-4-phénylthiazole ainsi que leurs sels, leur procédé de préparation et les compositions pharmaceutiques les contenant.

Les composés de la présente invention se distinguent d'autres dérivés du 1,3-thiazole par leur structure originale et par leur propriétés pharmacologiques inattendues. En effet, les composés de l'invention ont une affinité pour le récepteur du fibrinogène : le complexe des glycoprotéines IIb/IIIa (GP IIb/IIIa) ; plus particulièrement la présente invention a pour objet des nouveaux inhibiteurs de l'agrégation plaquettaire antagonistes des récepteurs GP IIb/IIIa, de structure non peptidique, actifs par voie parentérale et orale.

L'activation de l'agrégation plaquettaire est associée à des états pathologiques tels que les troubles du système cardiovasculaire et cérébrovasculaire comme dans les troubles thromboemboliques associés à l'athérosclérose et au diabète tels l'angine instable, l'attaque cérébrale, la resténose après angioplastie, endartérectomie ou pose de stents, à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires, de pontages aorto coronariens mais aussi à l'ostéoporose, au cancer et au métastases ou aux glomérulonéphrites.

La contribution des plaquettes à ces processus pathologiques est due à leur capacité de former des agrégats ou caillots plaquettaires, en particulier sur les parois artérielles lésées suite à une rupture de la plaque athéromateuse.

Les plaquettes sont connues pour jouer un rôle essentiel dans le maintien de l'hémostase et dans les pathogénèses de la thrombose artérielle. Il a été démontré que l'activation des plaquettes augmente pendant la thrombolyse coronarienne pouvant affecter la reperfusion et entraîner la réocclusion de l'artère.

Des études cliniques réalisées avec l'aspirine et la ticlopidine ont démontré que l'inhibition de l'agrégation plaquettaire était un moyen efficace de prévenir les accidents cardiovasculaires dans plusieurs types de populations à risque.

Les plaquettes sont activées par un grand nombre d'agonistes ce qui a pour conséquence la modification de leur forme, ainsi que la modification de la sécrétion du contenu granulaire et l'agrégation. L'agrégation des plaquettes contribue ensuite à la formation des caillots.

Plusieurs agonistes endogènes tels que l'adénosine-5'-diphosphate (ADP), la sérotonine, l'acide arachidonique, le facteur d'activation plaquettaires (PAF), l'adrénaline, la thrombine ou le collagène ont été identifiés.

Compte tenu de l'implication simultanée de plusieurs agonistes endogènes dans l'activation des fonctions plaquettaires et de l'agrégation, un inhibiteur qui agirait contre tous les agonistes représenterait un agent antiplaquettaire plus efficace que les médicaments actuellement disponibles qui sont des produits agissant spécifiquement contre un agoniste particulier.

Les antiagrégants plaquettaires disponibles aujourd'hui sont actifs seulement contre un type d'agoniste. C'est le cas de l'aspirine, active vis-à-vis de l'acide arachidonique, de la ticlopidine active vis-à-vis de l'ADP, des inhibiteurs de thromboxane A₂ synthétase ou des antagonistes du récepteur du thromboxane A₂ ou encore de l'hirudine agissant contre la thrombine.

Récemment, un mode d'action commun à tous les agonistes connus a été mis en évidence. Il s'agit de l'activation du complexe membranaire des glycoprotéines GP IIb/IIIa qui fixe alors le fibrinogène circulant réalisant ainsi le pontage entre plusieurs plaquettes et donc l'agrégation plaquettaire. De récentes revues ont montré l'intérêt de produits antagonistes de la GP IIb/IIIa, par exemple Drugs of the Future, 1994, 19 (2), 135-159 ; ou 19 (5), 461-476 ou encore 19 (8), 757-764.

La GP IIb/IIIa de plaquettes non stimulées ne se lie pas aux protéines solubles. En revanche, la GP IIb/IIIa de plaquettes activées est connue pour se lier à des protéines adhésives telles que le fibrinogène, le facteur de von Willebrand, la fibronectine, ou la vitronectine. La liaison du fibrinogène et du facteur de von Willebrand à la GP IIb/IIIa entraîne une agrégation plaquettaire. La liaison du fibrinogène est médiée en partie par la séquence de reconnaissance Arg-Gly-Asp (RGD) commune aux protéines adhésives qui se fixent à la GP IIb/IIIa (Thromb. Res. 1993, 72, 231-245).

Des antagonistes de GP IIb/IIIa ont déjà fait l'objet de demandes de brevet ; c'est le cas par exemple de EP-608858, EP-542363, EP-539343, EP-478363, EP-623595, WO 93/14077, WO 94/22910 et depuis quelques années, les propriétés antithrombotiques d'inhibiteurs de GP IIb/IIIa ont été décrites (voir Drugs of the Future cité plus haut). Parmi les composés étudiés un anticorps monoclonal le c7E3 (Abciximab) a présenté d'intéressantes propriétés antithrombotiques en clinique humaine décrites notamment dans N. Engl., J. Med., 1994, *330*, 956-61. Ces résultats sont d'un intérêt particulier puisqu'ils montrent que de tels produits sont de bons candidats dans la prévention de la thrombose et de ses complications. Ce produit nécessite une administration par voie intraveineuse. Néanmoins, parmi les inhibiteurs de GP IIb/IIIa étudiés à ce jour chez l'homme, certains semblent présenter une activité par voie orale.

C'est notamment le cas pour des composés tels que SC 54684, L 703-014, GR 144053, DMP 728, ou encore BIBU-104 (Zablocki, J.A. et al., Exp. Opin. Invest. Drugs., 1994, 3, *5*, 449-55).

Le développement d'antagonistes de GP IIb/IIIa actifs par voie orale constitue donc une nouvelle approche prometteuse pour la thérapie des pathologies dépendantes de l'agrégation plaquettaire.

Il existe aujourd'hui un besoin qui consiste à pouvoir disposer d'un antiagrégant plaquettaire spécifique de la GP IIb/IIIa qui inhiberait l'activation et l'agrégation des plaquettes quel que soit l'agoniste impliqué ; celui-ci devrait être actif par voie orale.

Un tel inhibiteur serait pourvu de propriétés thérapeutiques vis-à-vis de l'agrégation plaquettaire plus efficaces que celles des antagonistes spécifiques disponibles actuellement.

Ainsi la présente invention concerne des dérivés du thiazole utiles comme antagonistes du complexe des glycoprotéines GP IIb/IIIa, les compositions pharmaceutiques qui les contiennent, l'utilisation de ces composés seuls ou en association avec d'autres agents antithrombotiques comme les anticoagulants et/ou les thrombolytiques, pour les traitements des troubles thromboemboliques et de toutes pathologies en dépendant.

La présente invention concerne également des associations de principe actif qui sont des compositions pharmaceutiques contenant au moins un composé selon l'invention en association avec des anticoagulants tels que la warfarine, l'héparine, les boropeptides, les hirudines ou l'argatroban ou des agents antiagrégants plaquettaires tels que l'aspirine, la ticlopidine ou encore des agents thrombolytiques comme l'activateur tissulaire du plasminogène, l'anistreplase, l'urokinase ou la streptokinase ou des associations de ceux-ci, les compositions comprenant ces mélanges étant utilisées pour inhiber l'agrégation plaquettaire et les troubles thromboemboliques.

Les composés selon la présente invention répondent à la formule suivante : 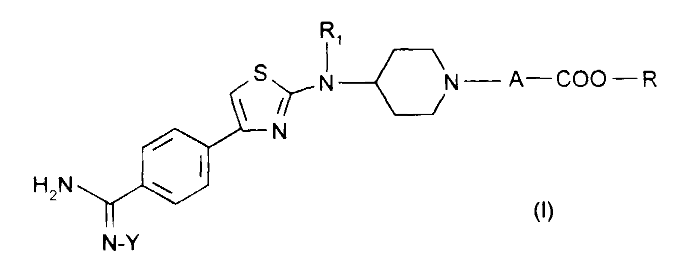 dans laquelle
- R₁ représente l'hydrogène, un alkyle en C₁-C₅, un cycloalkyle en C₃-C₈, un aralkyle dont la partie alkyle est en C₁-C₅, un groupe alcoxycarbonylalkyle ou un groupe (alcoxycarbonylaryl)alkyle dans lesquels les parties alcoxy et alkyle sont en C₁-C₃ ; un groupe carboxyalkyle ou un groupe (carboxyaryl)alkyle dans lesquels la partie alkyle est en C₁-C₃ ;
- A représente soit (i) un groupe méthylène éventuellement mono ou di-substitué par un groupe alkyle en C₁-C₅ ; par un groupe alcoxycarbonyle dont la partie alcoxy est en C₁-C₅ ; par un groupe alcoxycarbonylalkyle dans lequel les parties alcoxy et alkyle sont en C₁-C₅ ; par un groupe carboxyalkyle dont la partie alkyle est en C₁-C₅ ; par un groupe choisi parmi le phényle et le benzyle non substitués ou substitués sur le noyau aromatique par un alkyle en C₁-C₅, un alcoxy en C₁-C₅, un hydroxyle, un halogène ou un trifluorométhyle ; par un groupe pyridyle ; soit (ii) un groupe éthylène ;
- R représente l'hydrogène; un groupe alkyle en C₁-C₅ ; un groupe aryle ou un groupe aralkyle dont la partie alkyle est en C₁-C₅, lesdits groupes aryle et aralkyle étant non substitués ou substitués sur le cycle aromatique par un hydroxyle, un alcoxy en C₁-C₃, un alcanoyloxy en C₁-C₃, un halogène, un trifluorométhyle ou un alkyle en C₁-C₅ ;
- Y représente l'hydrogène ; un groupe -COOR₂ dans lequel R₂ représente un groupe alkyle en C₁-C₅, un groupe aryle ou un groupe aralkyle dont la partie alkyle est en C₁-C₅ ,lesdits groupes aryle et aralkyle pouvant être éventuellement substitués sur le noyau aromatique par un alkyle en C₁-C₅ ; ou un groupe -COR₃ dans lequel R₃ représente un alkyle en C₁-C₅ ;
ou un de leurs sels.

Selon l'invention on entend notamment par aryle, un noyau aromatique par exemple en C₆-C₁₀, notamment phényle, 1-naphtyle ou 2-naphtyle.

De même, la partie aryle des groupes aralkyle, (alcoxy carbonylaryl)alkyle et (carboxyaryl)alkyle selon l'invention représente de préférence un noyau aromatique par exemple en C₆-C₁₀, notamment phényle, 1-naphtyle ou 2-naphtyle.

Les sels des composés de formule (I) selon la présente invention comprennent aussi bien ceux obtenus avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que les sels pharmaceutiquement acceptables tels que le chlorhydrate, le bromhydrate, le sulfate, l'acétate, l'hydrogénosulfate, le dihydrogénophosphate, le méthanesulfonate, le méthylsulfate, le maléate, le fumarate, le sulfonate, le 2-naphtalènesulfonate, le glycolate, le gluconate, le citrate, l'iséthionate, le benzoate, le salicylate, l'ascorbate, le tartrate, le succinate, le lactate, le glutarate, le toluène sulfonate, l'ascorbate ou encore les sels de base inorganiques sous forme de sels alcalins métalliques tels que par exemple les sels de sodium.

La présente invention concerne également les isomères optiques des composés de formule (I) qui contiennent éventuellement un centre chiral, ainsi que leurs sels.

Les énantiomères et les mélanges racémiques des composés de formule (I) sont donc partie intégrante de l'invention.

Dans la présente description les groupes alkyles ou les groupes alcoxy sont droits ou ramifiés. Les groupes alkyles sont par exemple méthyle, éthyle, *n*-propyle, *i*-propyle, *n*-butyle, *sec*-butyle ou *t*-butyle et les groupes alcoxy sont par exemple méthoxy, éthoxy, *n*-propoxy, *i*-propoxy, *n*-butoxy, *sec*-butoxy ou *t*-butoxy.

Les composés de formule (I) dans lesquels R représente méthyle ou éthyle sont préférés.

Un autre groupe de composés préférés est constitué par ceux de formule : 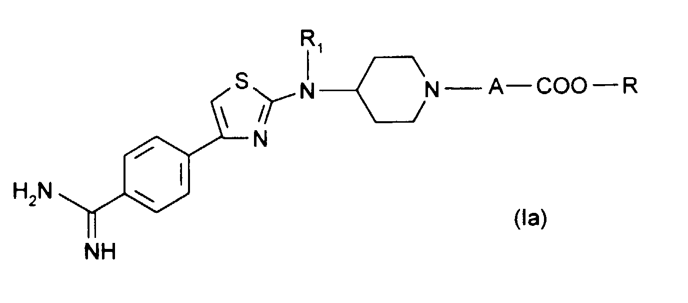 dans laquelle -A- représente un groupe méthylène éventuellement mono substitué, R₁ représente l'hydrogène, un groupe méthyle, un groupe carboxyalkyle ou un groupe alcoxycarbonylalkyle et -COO-R est tel que défini pour (I), ou un de leurs sels.

Un autre groupe de composés préférés est constitué par les composés de formule : 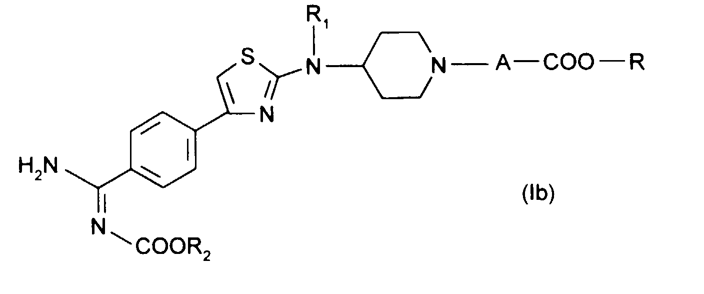 dans laquelle -A- représente un groupe méthylène éventuellement mono substitué et COO-R, R₁ et R₂ sont tels que définis pour (I), ou un de leurs sels.

Particulièrement préféré est le groupe de composés de formule : 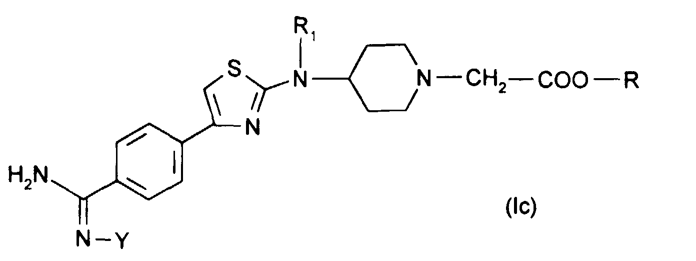 dans laquelle R, Y et R₁ sont tels que définis pour (I) ou un de leurs sels.

Plus particulièrement préféré est le groupe de composés de formule : 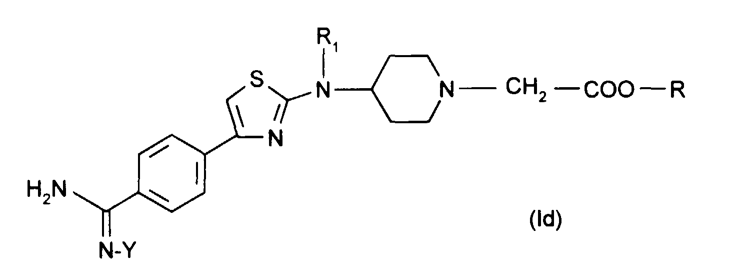 dans laquelle R₁ représente l'hydrogène, un groupe carboxyalkyle ou un groupe alcoxycarbonylalkyle, R représente l'hydrogène, un groupe méthyle ou éthyle et Y est tel que défini pour (I) ou un de leurs sels.

Les composés plus particulièrement préférés sont :
a) (4-{4-[4-(aminoiminométhyl)-phényl]-1,3-thiazol-2-yl-amino}-pipéridin-1-yl)- acétate de méthyle,
b) acide (4-{4-[4-(aminoiminométhyl)-phényl]-1,3-thiazol-2-yl-amino}-pipéridin-1-yl)-acétique,
c) (4-{4-[4-(amino(N-éthoxycarbonyl-imino)méthyl)-phényl]-1,3-thiazol-2-yl-amino}-pipéridin-1-yl)-acétate de méthyle,
d) (4-{4-[4-(aminoiminométhyl)-phényl]-1,3-thiazol-2-yl-amino}-pipéridin-1-yl)- acétate d'éthyle,
e) (4-{4-[4-(amino(N-éthoxycarbonyl-imino)méthyl)-phényl]-1,3-thiazol-2-yl-amino}-pipéridin-1-yl)-acétate d'éthyle,
f) acide (4-{4-[4-(aminoiminométhyl)-phényl]-1,3-thiazol-2-yl-N-carboxyméthyl-amino}-pipéridin-1-yl)-acétique,
g) 3-[N-{4-[4-(aminoiminométhyl)-phényl]-1,3-thiazol-2-yl}-N-(1-éthoxycarbonyl-méthylpipéridin-4-yl)-amino]-propionate d'éthyle,
h) acide 3-[N-{4-[4-(aminoiminométhyl)-phényl]-1,3-thiazol-2-yl}-N-(1-carboxy- méthylpipéridin-4-yl)-amino]-propionique,
i) 3-[N-{4-[4-(aminoéthoxycarbonyliminométhyl)-phényl]-1,3-thiazol-2-yl}-N-(1-éthoxycarbonylméthylpipéridin-4-yl)-amino]-propionate d'éthyle, ou un de leurs sels.

La présente invention concerne également un procédé pour la préparation des composés (I) à partir des composés intermédiaires de formule (II).

Ce procédé comprend les étapes consistant à :
(a) déprotéger la fonction amine cyclique d'un composé de formule (II): 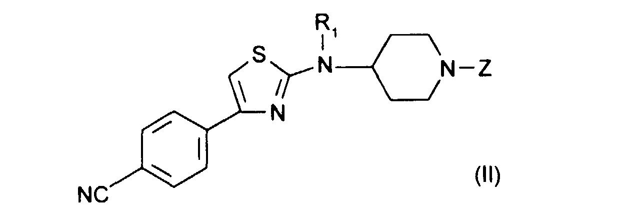 dans laquelle R₁ est tel que défini pour (I) et Z représente un groupe protecteur d'une fonction amine tel qu'un benzyle, de façon à obtenir une amine libre de formule (III) : 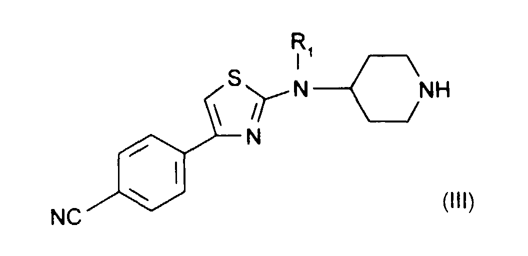 dans laquelle R₁ est tel que défini pour (I);
   lorsque Z représente un benzyle, la déprotection peut être réalisée par action de chloroformiates ;
(b) N-alkyler le composé résultant de formule (III) (i) soit par réaction avec un dérivé halogéné de formule :

   X-A-COO-R (1)

   dans lequel X représente un groupe nucléofuge tel qu'un groupe tosyle ou un halogène de préférence le chlore ou le brome et A et R sont tels que définis pour (I), dans un solvant choisi par exemple parmi les alcanols ou le diméthylformamide en présence d'un agent alcalin tel qu'un carbonate alcalin ou la triéthylamine ;
   (ii) soit, par réaction de Michaël avec un ester α, β-insaturé de formule

   CH₂ = CH-COOR (2)

   ou

   RCOO-CH=CH-COOR (3)

   dans un alcanol où R est tel que défini pour (I),
   pour obtenir un composé de formule : 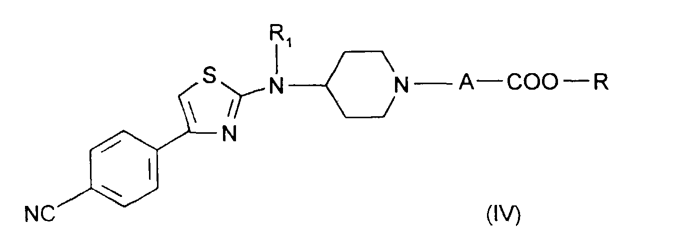 dans lequel A, R et R₁ sont tels que définis pour (I),
(c) soumettre le composé résultant à la réaction de Pinner en faisant réagir le nitrile (IV) en milieu acide dans un alcanol pour obtenir le sel imidate sur lequel on fait ensuite réagir l'ammoniac pour obtenir l'amidine de formule : 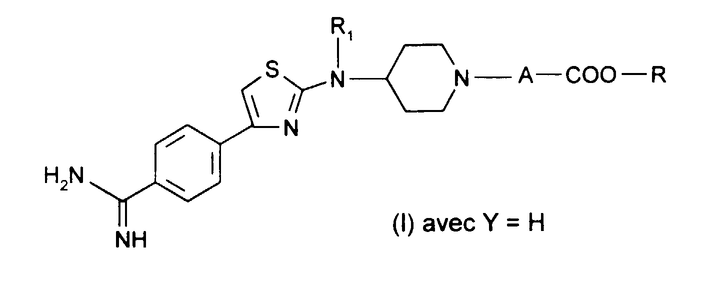 dans laquelle A, R et R₁ sont tels que définis pour (I), le composé obtenu réprésentant un composé de formule (I) dans laquelle Y est l'hydrogène,
(d) le cas échéant, à faire réagir l'amidine obtenue à l'étape (c)
   (i) soit avec un chloroformiate de formule :

      Cl-COO-R₂

      dans lequel R₂ est tel que défini pour (I), dans un solvant tel que le diméthylformamide en milieu alcalin par exemple en présence de triéthylamine ou d'un carbonate alcalin pour obtenir le composé de formule : 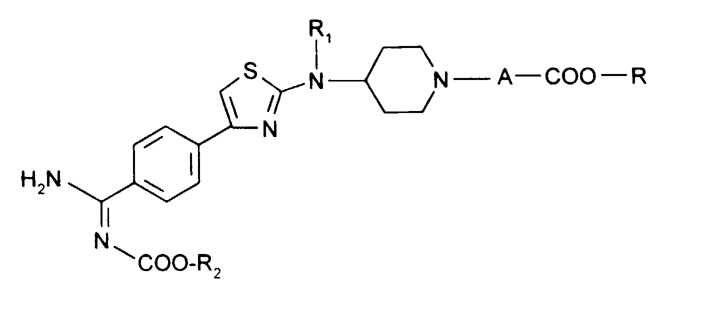 représentant un composé de formule (I) dans lequel Y représente -COO-R₂ et R₁, R₂, A et R sont tels que définis pour (I) ;
   (ii) soit avec un agent acylant tel que Cl-CO-R₃ dans lequel R₃ est tel que défini pour (I) pour obtenir un composé de formule (I) dans lequel Y représente -CO-R₃, R₁, A et R étant tels que définis pour (I) ;
(e) le cas échéant, à hydrolyser le précurseur ester correspondant obtenu à l'étape (c), par exemple en milieu acide en présence d'acide chlorhydrique pour obtenir un composé de formule (I) dans lequel R est hydrogène, ou un de leurs sels.

Les composés intermédiaires de formule (II) dans lesquels R₁ est différent de l'hydrogène peuvent être préparés simplement à partir des composés correspondants de formule (II) dans lesquels R₁ est un atome d'hydrogène par action d'un dérivé de formule R₁ X dans lequel R₁ est tel que défini pour (I) et X est un groupe nucléofuge tel qu'un groupe alkylsulfonyloxy, arylsulfonyloxy ou un atome d'halogène, en présence soit d'une base forte telle qu'un hydrure alcalin par exemple l'hydrure de sodium, ou telle que le *tert*-butylate de potassium dans un solvant anhydre tel que le diméthylformamide ou le tétrahydrofurane, soit par catalyse par transfert de phase (CTP) solide-liquide en présence d'un catalyseur de transfert de phase par exemple le bromure de tétrabutylammonium, et d'une base comme le carbonate de potassium dans un solvant inerte tel que le toluène.

Dans le cas où R₁ = -CH₂-CH₂-COOR₄ (avec R₄ représentant un hydrogène ou un groupe alkyle en C₁-C₂), les composés de formule (II) dans lesquels R₁ = -CH₂-CH₂-COOR₄ peuvent être avantageusement obtenus à partir des composés correspondants de formule (II) dans lesquels R₁ est un atome d'hydrogène, par réaction de type Michaël avec un composé α, β-insaturé de formule CH₂ = CH-COOR₄ (R₄ représentant un atome d'hydrogène ou un groupe alkyle en C₁-C₂) dans des conditions de CTP solide-liquide en présence d'un catalyseur comme le bromure de tétrabutylammonium et d'une base comme le carbonate de potassium dans un solvant inerte tel que le toluène.

Les composés intermédiaires (II) dans lesquels R₁ représente l'hydrogène sont préparés selon des méthodes bien connues de l'homme de l'art et notamment selon la méthode de Hantzsch, Comprehensive Heterocyclic Chemistry, A. Katritzky, 1984, Vol 6, à partir de produit de départ disponibles commercialement ou préparés selon des méthodes connues.

Les composés de formule (II), (III) et (IV) et leurs sels sont des composés originaux qui constituent un autre aspect de l'invention.

Ils peuvent être représentés par la formule (V) suivante : 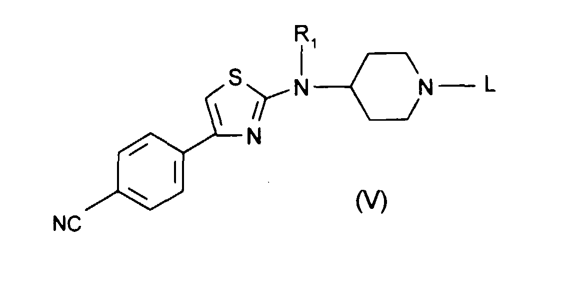 dans laquelle R₁ est tel que défini ci-dessus et L représente l'hydrogène, un groupe Z ou un groupe -A-COOR, Z, A et R étant tels que définis ci-dessus.

Les composés de formule (I) ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligand de récepteurs.

Le SCHEMA 1 suivant rapporte l'ensemble des réactions discutées ci-dessus :

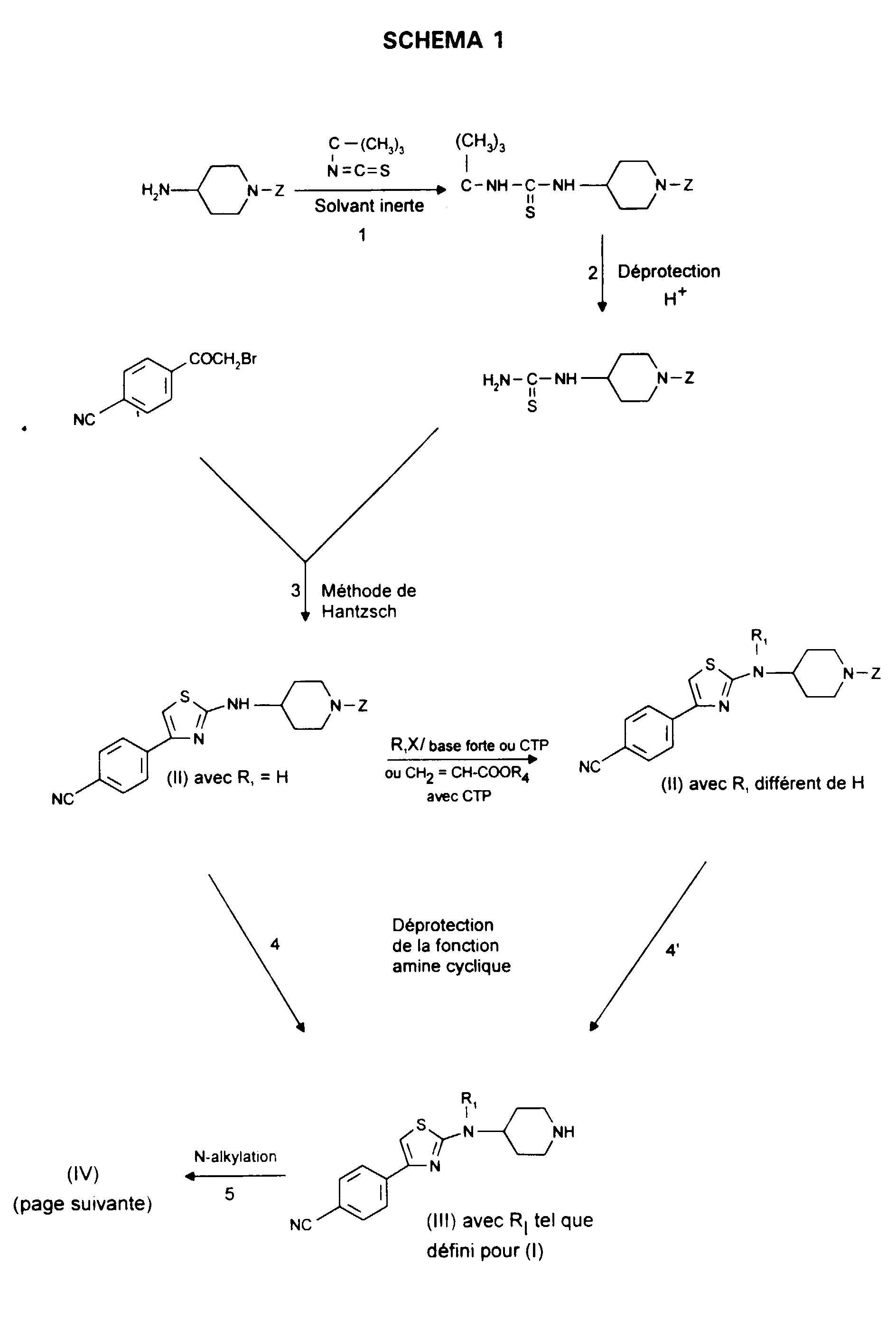

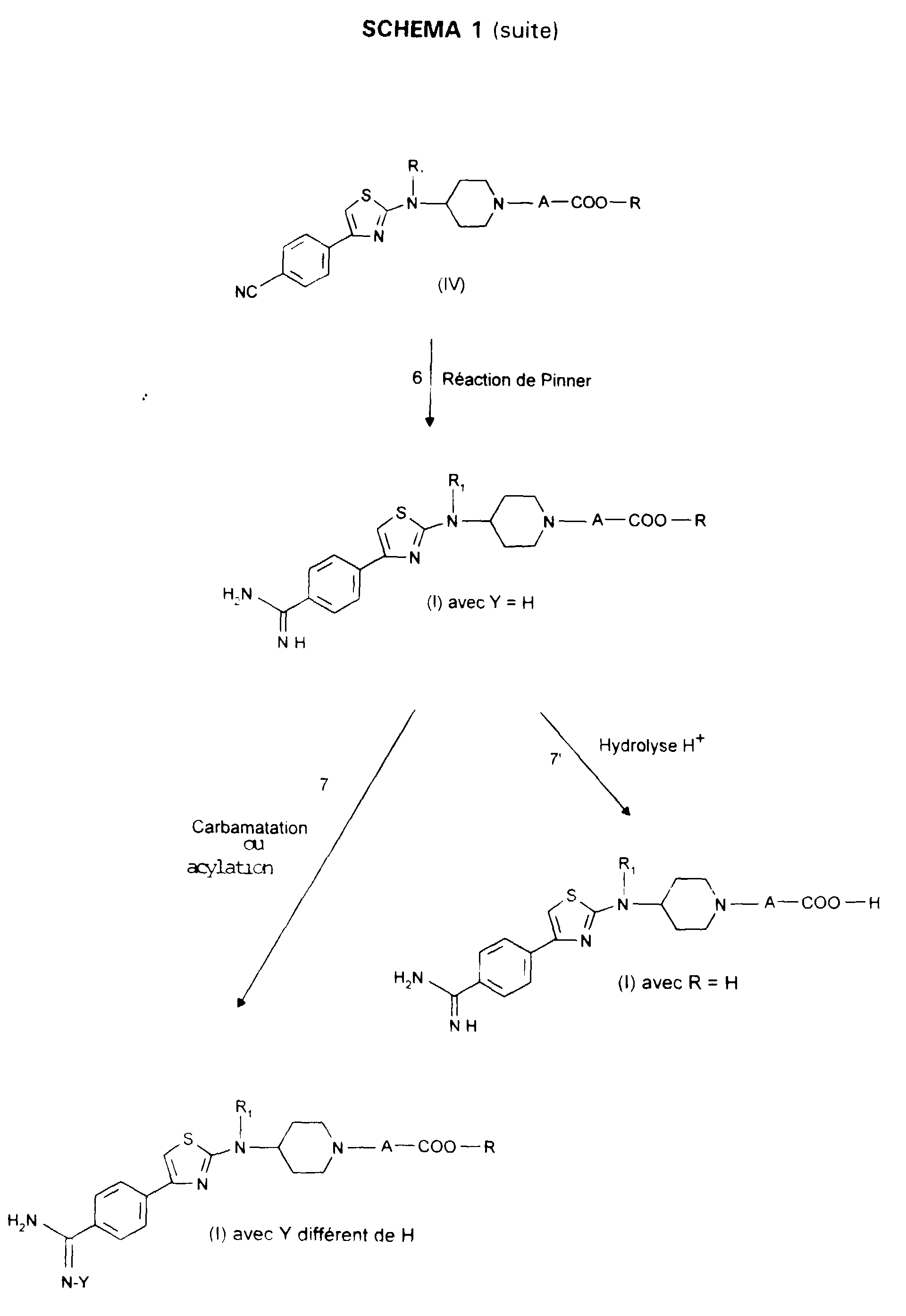

Compte tenu de leur activité antagoniste de la fixation du fibrinogène aux plaquettes, les composés de la présente invention sont très utiles en médecine humaine et vétérinaire, en particulier pour le traitement et la prophylaxie des désordres thrombotiques. De nombreux exemples de désordres thrombotiques sont connus dans la littérature et comprennent notamment les maladies vasculaires occlusives tels que l'infarctus du myocarde, les angines de poitrine, les accidents ischémiques transitoires, les attaques cérébrales d'origine thrombotiques, l'athérosclérose, les maladies artérielles périphériques, les néphropathies, les rétinopathies, les thromboses post opératoires, les embolies pulmonaires, les thromboses veineuses et dans l'angioplastie.

Les composés selon l'invention sont également intéressants et utiles dans la prophylaxie des complications péri et post opératoires consécutives à une transplantation d'organe, en particulier du coeur et du rein.

Les composés selon l'invention sont également utiles pour le traitement ou la prophylaxie d'autres pathologies dans lesquelles le complexe GP IIb/IIIa ou d'autres récepteurs (intégrine) sont impliqués. Ainsi, par exemple, les composés de l'invention peuvent être utilisés pour potentialiser la cicatrisation, pour le traitement de l'ostéoporose ou de la polyarthrite rhumatoïde.

Les composés selon l'invention peuvent également être utiles pour le traitement de certaines maladies cancéreuses et sont utiles pour prévenir ou arrêter le développement de métastases cancéreuses.

Un autre aspect de l'invention concerne les sels pharmaceutiquement acceptables des composés selon l'invention pour leur usage dans le traitement ou la prophylaxie des désordres thrombotiques, ainsi que la préparation d'un médicament en contenant.

Les composés selon l'invention ont fait l'objet d'essais biochimiques et pharmacologiques.

L'activité pharmacologique des composés (I) selon l'invention a été évaluée par des essais d'agrégation plaquettaire. Ces essais ont été réalisés sur des échantillons provenant d'homme, de babouin, de chien, de cobaye, de lapin, de rat et de souris et ont permis de déterminer l'activité *in vitro* des composés. L'activité *ex vivo* de ces produits, administrés par voie intraveineuse ou orale, a aussi été mesurée chez le babouin par la même méthode.

L'agrégation plaquettaire a été induite selon l'un des deux protocoles suivants :

**Protocole d'agrégation plaquettaire par turbidimétrie selon G.V. Born, Agregation of blood platelets by adenosine diphosphate and its reversal, Nature, 1962, *194*, 927-929 modifié selon SAVI et al., Nouv. Rev. Fr. Hematol, 1993, *35*, 115-119.**

L'agrégation plaquettaire (humain, chien, cobaye, lapin, rat, souris et babouin) a été mesurée par turbidimétrie sur 400 µl de plasma riche en plaquettes (PRP) obtenu par centrifugation à partir de sang total prélevé sur une solution de citrate trisodique à 3,8 % (9 vol / 1 vol). 400 µl de PRP sont déposés dans la cuve d'un agrégomètre, sous agitation (900 tours/min), à 37°C. L'induction de l'agrégation se fait par ajout de 4 µl d'une solution d'agent agrégant.

**Protocole d'agrégation en sang total selon Diodati et al., Circulation, 1992, *86*, 1186-1193.**

L'agrégation plaquettaire (chien) est mesurée par impédance sur du sang total, prélevé sur une solution d'hirudine à 100 µg/ml (9 vol / 1 vol). 0,5 ml de sang total ainsi prélevé et 0,5 ml d'une solution de NaCl à 0,9 % sont déposés dans la cuve de l'agrégomètre, sous agitation, à 37°C puis l'électrode est trempée dans la cuve. L'agrégation est mesurée après activation par 10 µl d'une solution d'agent agrégant.

Les agents agrégants étudiés ont été l'ADP (2,5 ou 6,25 µM), l'acide arachidonique (500 µM) le collagène (12,5 µg/ml), la thrombine (0,1 Ul/ml), ou le PAF (0,5 µM). Dans le cas des tests *in vitro*, 10 µl d'une solution de DMSO à 10 % contenant les composés de l'invention ont été introduits dans la cuve 30 secondes avant le déclenchement de l'agrégation. Les contrôles ont été réalisés en présence d'un même volume de solvant.

Les composés selon l'invention présentent une activité vis-à-vis de l'agrégation plaquettaire à une concentration comprise entre 1 nM et 10 µM et ce quel que soit l'agent agrégant utilisé.

Après administration orale ou intraveineuse, une activité pharmacologique a pu être observée chez le babouin pour certains composés administrés par voie intraveineuse ou orale, à des doses variant de 0,01 à 100 mg/kg.

Les composés de formule (I) sont peu toxiques ; leur toxicité est compatible avec leur utilisation comme médicament pour le traitement des troubles et des maladies ci-dessus.

Les composés de formule (I), peuvent être formulés dans des compositions pharmaceutiques pour l'administration aux mammifères, y compris l'homme, pour le traitement des maladies susdites.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,01 à 5 mg/kg.

Chez l'être humain, la dose peut varier de préférence de 0,1 à 500 mg par jour, plus particulièrement de 2 à 500 mg selon l'âge du sujet à traiter ou le type de traitement : prophylactique ou curatif.

Dans les compositions pharmaceutiques de la présente invention, le principe actif est généralement formulé en unités de dosage contenant de 0,01 à 500 mg, avantageusement de 0,2 à 500 mg, de préférence de 0,2 à 200 mg dudit principe actif par unité de dosage.

La présente invention a donc également pour objet les compositions pharmaceutiques qui contiennent à titre de principe actif un des composés ci-dessus. Ces compositions sont réalisées de façon à pouvoir être administrées par la voie orale ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou γ cyclodextrine, 2-hydroxypropyl-β-cyclodextrine ou méthyl-β-cyclodextrine.

Les PREPARATIONS et les EXEMPLES suivants illustrent l'invention sans toutefois la limiter.

### Préparation des intermédiaires de formules (II), (III) et (IV)

### PREPARATION I

### N-(1-Benzylpipéridin-4-yl)-N'-tert-butylthiourée (Composé 1)

On dissout 24,93 g de 4-amino-1-benzylpipéridine dans 300 ml de dichlorométhane puis on ajoute, goutte à goutte, et à température ambiante, 16,7 ml de *tert*-butylisothiocyanate. Le mélange réactionnel est agité à température ambiante pendant 5 heures puis on ajoute de l'eau et on laisse décanter. La phase organique est séparée, séchée sur sulfate de sodium et évaporée. Le résidu obtenu est cristallisé dans l'éther de pétrole pour fournir des cristaux blancs qui fondent à 137°C ; Rendement : 88 %.

### PREPARATION II

### Bromhydrate de N-(1-benzylpipéridin-4-yl)thiourée (Composé 2)

A 170 ml d'acide bromhydrique à 33 % dans l'acide acétique on ajoute 17 g de N-(1-benzylpipéridin-4-yl)-N'-*tert*-butylthiourée, on agite le mélange réactionnel à température ambiante pendant 4 heures, puis on le verse sur de l'éther diéthylique. On filtre les cristaux obtenus et on les lave abondamment à l'éther diéthylique pour obtenir des cristaux blancs qui fondent à 120°C ; Rendement : quantitatif.

### PREPARATION III

### Dibromhydrate de 4-{2-[N-(1-benzylpipéridin-4-yl)-amino]-1,3-thiazol-4-yl}-benzonitrile (Composé 3, (II) avec R₁ = H))

A 18,38 g de bromhydrate de N-(1-benzylpipéridin-4-yl)thiourée dans 200 ml de méthanol, on ajoute 12,46 g de bromure de 4-cyanophénacyle puis on chauffe le mélange réactionnel à reflux pendant 4 heures. On refroidit à température ambiante puis, successivement, on ajoute 200 ml d'éther diéthylique, filtre et lave le précipité à l'éther diéthylique pour obtenir des cristaux blancs qui fondent à 280°C ; Rendement : 86 %.

### PREPARATION IV

### 4-{2-[N-(1-Benzylpipéridin-4-yl)-N-méthylamino]-1,3-thiazol-4-yl}-benzonitrile (Composé 4.1, (II) avec R₁ = CH₃)

A 146 mg d'hydrure de sodium (à 60 % en dispersion dans l'huile) en suspension dans 13 ml de N,N-diméthylformamide, on ajoute, à température ambiante, 1,3 g de 4-{2-[N-(1-benzylpipéridin-4-yl)amino]-1,3-thiazol-4-yl} benzonitrile dissous dans 5 ml de N,N-diméthylformamide et on agite le mélange réactionnel pendant 15 minutes à cette température. On ajoute ensuite 0,23 ml d'iodure de méthyle, on agite le mélange réactionnel pendant 4 heures à température ambiante, puis on le verse sur de l'eau et on extrait à l'acétate d'éthyle. On lave la phase organique à l'eau, on sèche sur sulfate de sodium et on évapore. Le résidu est purifié par chromatographie sur gel de silice en éluant avec du dichlorométhane. On obtient des cristaux jaunes qui fondent à 136°C ; Rendement : 67 %.

### Oxalate de 3-{N-(1-Benzylpipéridin-4-yl)-N-[4-(4-cyanophényl)-1,3-thiazol-2-yl]-amino}-propionate d'éthyle (Composé 4.2, (II) avec R₁ = -CH₂ -CH₂-COOC₂H₅)

A 44,8 g de 4-{2-[N-(1-benzylpipéridin-4-yl)-amino]-1,3-thiazol-4-yl}-benzonitrile dans 900 ml de toluène, on ajoute 25,92 ml d'acrylate d'éthyle, 32,98 g de carbonate de potassium et 7,7 g de bromure de tétrabutylammonium, puis on chauffe le mélange réactionnel à reflux pendant 24 heures. Le mélange réactionnel est refroidi, puis on ajoute de l'eau et on décante. La phase organique est séchée sur sulfate de sodium puis évaporée à sec. L'huile obtenue est reprise par de l'éther isopropylique et filtrée sur un lit de silice en entrainant à l'éther isopropylique. L'évaporation du filtrat conduit à une résine qui est ensuite salifiée par ajout d'un équivalent d'acide oxalique dans l'acétone pour fournir des cristaux blancs qui fondent à 225°C ; rendement 84 %.

### PREPARATION V

### Dichlorhydrate de 4-{2-[N-(pipéridin-4-yl)-amino]-1,3-thiazol-4-yl}-benzonitrile (Composé 5.1, (III) avec R₁ = H)

Ce composé est obtenu par action de chloroformiates ainsi que décrit dans Tetrahedron Letters, 1983, *24*, 31, 3233-3236 et plus particulièrement par action de chloroformiate de 1-chloroéthyle selon J. Org. Chem., 1984, *49*, 2081-2082.

A 18 g de 4-{2-[N-(1-benzylpipéridin-4-yl)-amino]-1,3-thiazol-4-yl}-benzonitrile dans 250 ml de 1,2-dichloroéthane on ajoute 6,2 g de 1,8-bis-diméthylaminonaphtalène puis on refroidit le mélange réactionnel à 0°C, on ajoute 10,4 ml de chloroformiate de 1-chloroéthyle puis on agite le mélange réactionnel pendant 30 minutes à cette température et on chauffe ensuite à reflux 3 heures. On évapore à sec et reprend le résidu par 250 ml de méthanol puis chauffe le mélange à reflux pendant 2 heures. On laisse refroidir le mélange réactionnel puis succesivement, on ajoute 250 ml d'éther diéthylique, filtre, lave à l'éther diéthylique et sèche pour obtenir des cristaux beiges qui fondent à 266°C ; Rendement 87 %.

### Dichlorhydrate de 4-{2-[N-(pipéridin-4-yl)-N-méthylamino]-1,3-thiazol-4-yl}-benzonitrile (Composé 5.2, (III) avec R₁ = CH₃)

Ce composé est obtenu selon le même mode opératoire que pour le composé 5.1, par débenzylation du 4-{2-[N-(1-benzylpipéridin-4-yl)-N-méthylamino]-1,3-thiazol-4-yl}-benzonitrile. On obtient des cristaux beiges qui fondent à 175°C ; Rendement 67 %.

Dichlorhydrate de 3-{N-[4-(4-cyanophényl)-1,3-thiazol-2-yl]-N-(pipéridin-4-yl)-amino}-propionate d'éthyle **(Composé 5.3, (III) avec** **R**_{**1**} **= -CH**_{**2**} **-CH**_{**2**}**-COOC**_{**2**}**H**_{**5**}**)**

Ce composé est obtenu selon le même mode opératoire que pour le composé 5.1 par débenzylation du 3-{N-(1-benzylpipéridin-4-yl)-N-[4-(4-cyanophényl)-1,3-thiazol-2-yl]-amino}-propionate d'éthyle. On obtient des cristaux blancs qui fondent à 140°C ; Rendement 70 %.

### PREPARATION VI

### {4-[4-(4-Cyanophényl)-1,3-thiazol-2-yl-amino]-pipéridin-1-yl}-acétate de méthyle (Composé 6.1, (IV)) avec R₁ = H, A = CH₂ et R = CH₃)

A 14,8 g de dichlorhydrate de 4-{2-[(pipéridin-4-yl)-amino]-1,3-thiazol-4-yl}-benzonitrile dans 150 ml de N,N-diméthylformamide on ajoute 17,75 g de carbonate de potassium et 4,3 ml de bromoacétate de méthyle et on chauffe le mélange réactionnel à 50°C pendant 2 heures. On le verse sur de l'eau, on extrait à l'acétate d'éthyle et on lave la phase organique à l'eau, puis on sèche sur sulfate de sodium et on concentre sous vide. Le résidu cristallise dans l'éther isopropylique pour fournir des cristaux beiges qui fondent à 172°C ; Rendement 86 %.

### 3-{N-[4-(4-Cyanophényl)-1,3-thiazol-2-yl]-N-(1-éthoxycarbonylméthylpipéridin-4-yl)-amino}-propionate d'éthyle (Composé 6.2, (IV)) avec R₁ = -CH₂-CH₂-COOC₂H₅, A = CH₂ et R = C₂H₅)

Ce composé est obtenu selon le même mode opératoire que pour le composé 6.1, par N-alkylation du dichlorhydrate de 3-{N-[4-(4-cyanophényl)-1,3-thiazol-2-yl]-N-(pipéridin-4-yl)-amino}-propionate d'éthyle avec du bromoacétate d'éthyle. On obtient une résine incolore ; Rendement 85 %
¹H RMN (DMSO-d₆)δ : 1,16 (m, 6H) ; 1,73 - 1,96 (m, 4H) ; 2,29 (m, 2H) ; 2,66 (m, 2H) ; 2,89 (m, 2H) ; 3,23 (s, 2H) ; 3,50 (m, 1H) ; 3,62 (m, 2H) ; 4,04 (m, 4H) ; 7,49 (s, 1H) ; 7,82 (d, J = 8,44 Hz, 2H) ; 7,98 (d, J = 8,44 Hz, 2H).

### PREPARATION VII

### 3-{4-[4-(4-Cyanophényl)-1,3 thiazol-2-yl-amino]-pipéridin-1-yl}-propionate de méthyle (Composé 7.1)

Ce composé est obtenu par réaction de Michaël avec un ester α, β-insaturé dans un alcanol, selon Organic Reaction, 1967, vol. 10, 173.

A 2 g de dichlorhydrate de 4-{2-[(pipéridin-4-yl)-amino]-1,3-thiazol-4-yl}-benzonitrile dans 30 ml de méthanol, on ajoute 1,64 ml de triéthylamine puis 0,55 ml d'acrylate de méthyle et chauffe le mélange réactionnel à reflux pendant 1 heure. Le mélange réactionnel est ensuite évaporé à sec. Le résidu est purifié par chromatographie sur gel de silice en éluant avec un mélange dichlorométhane-méthanol (95/5). La concentration des fractions de produit pur fournit des cristaux blancs qui fondent à 154°C ; Rendement : 72 %.

En procédant selon les PREPARATIONS VI et VII décrites ci-dessus, on prépare les Composés 6.3 à 7.2 décrits dans le TABLEAU I ci-après :

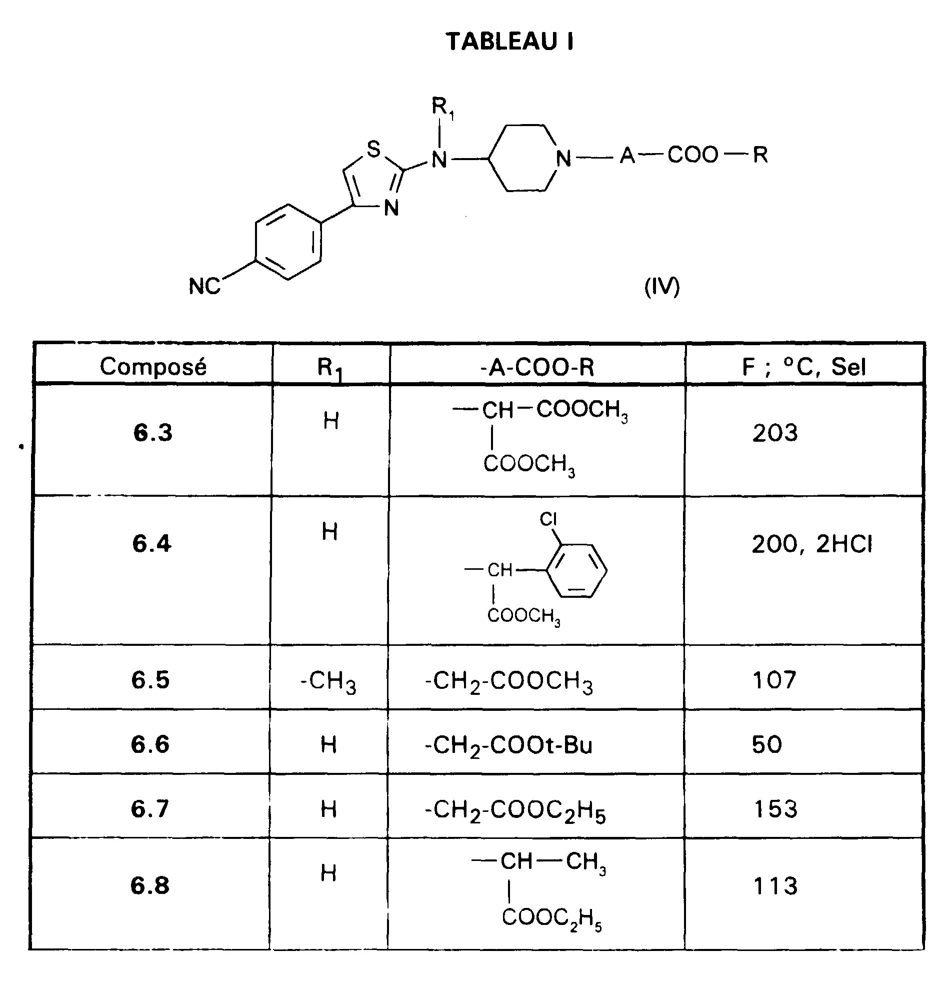

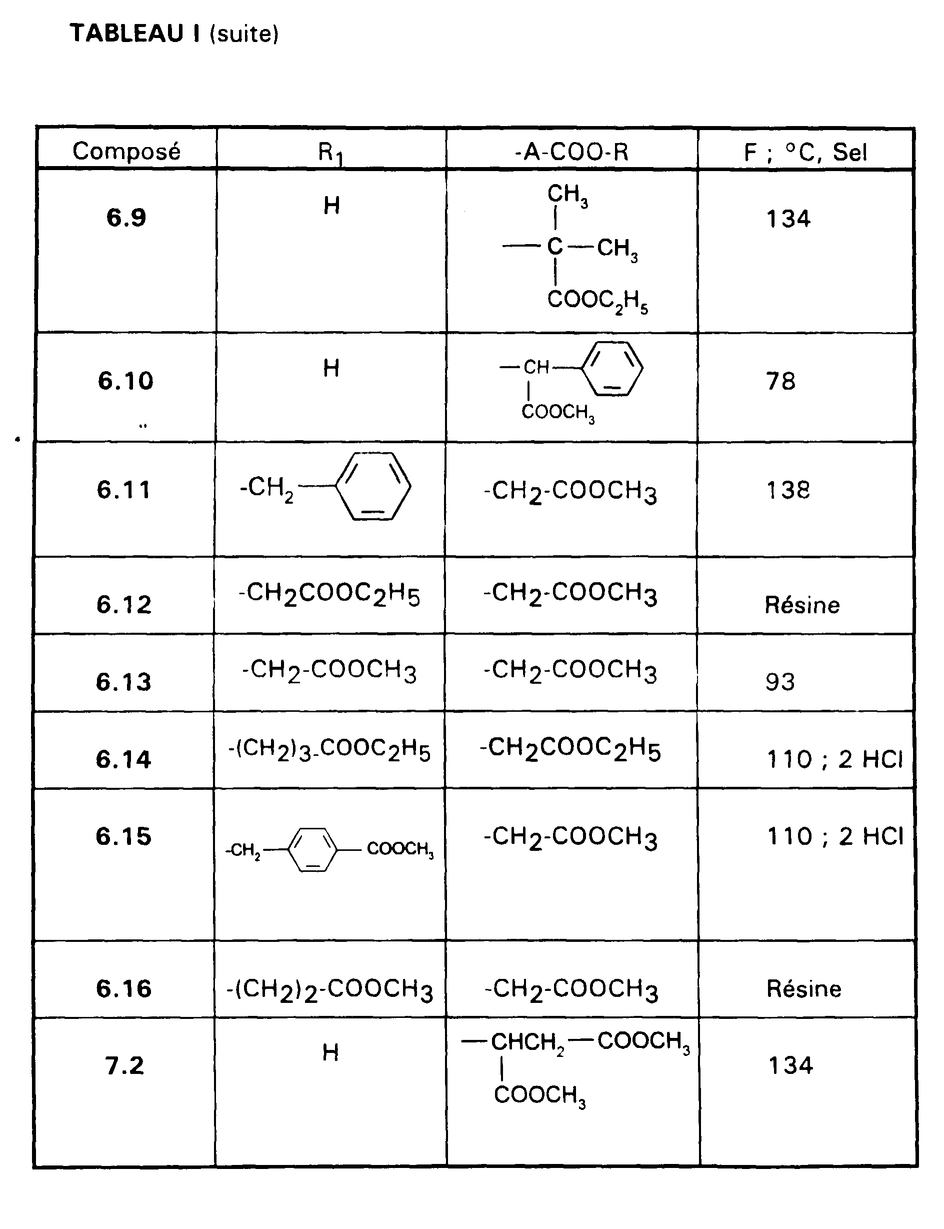

### EXEMPLE 1

### Chlorhydrate de (4-{4-[4-(aminoiminométhyl)phényl]-1,3-thiazol-2-yl-amino}-pipéridin-1-yl)-acétate de méthyle

(I) : Y = H ; -A-COOR = -CH₂COOCH₃ ; R₁ = H

Ce composé, ainsi que celui de l'EXEMPLE 2 ci-après, est obtenu par la réaction de Pinner selon Organic Functional Group Preparation, Sandler S. and Karo W., W.,1989, 12, *III*, Second Edition.

A 150 ml de méthanol saturé en gaz chlorhydrique à une température voisine de 0°C on ajoute 10,33 g de {4-[4-(4-cyanophényl)-1,3-thiazol-2-yl-amino}-pipéridin-1-yl]-acétate de méthyle (Composé 6.1) et on laisse le mélange réactionnel à 4°C pendant une nuit. On évapore à sec sans chauffer puis on reprend le résidu par 150 ml de méthanol et on fait barbotter de l'ammoniac jusqu'à pH basique. On chauffe ensuite le mélange réactionnel à reflux pendant 2 heures, puis on évapore à sec et on purifie le résidu obtenu par chromatographie sur colonne de gel de silice en éluant avec un mélange dichlorométhane-méthanol (8/2 - v/v). La concentration de fraction de produit pur fournit des cristaux jaunes qui fondent à 150°C ; Rendement : 69 %.

### EXEMPLE 2

### Chlorhydrate de 3-[N-{4-[4-(aminoiminométhyl)-phényl]-1,3-thiazol-2-yl}-N-(1-éthoxycarbonylméthylpipéridin-4-yl)-amino]-propionate d'éthyle.

(I) : Y = H ; -A-COOR = -CH₂-COOC₂H₅ ; R₁ = -CH₂-CH₂-COOC₂H₅

Une solution de 1,25 g de 3-{N-[4-(4-Cyanophényl)-1,3-thiazol-2-yl]-N-(1-éthoxycarbonylméthyl-pipéridin-4-yl)-amino}-propionate d'éthyle (Composé 6.2) dans 25 ml d'éthanol est saturée à 0°C en gaz chlorhydrique ; on laisse le mélange réactionnel à 4°C pendant une nuit puis on évapore à sec sans chauffer et successivement on reprend le résidu par 40 ml d'éthanol et on fait barbotter un courant d'ammoniac jusqu'à pH basique. On chauffe ensuite le mélange réactionnel à reflux pendant deux heures, on évapore à sec puis on purifie le résidu obtenu par chromatographie sur colonne de gel de silice en éluant avec un mélange dichlorométhane-méthanol (8/2 - v/v). La concentration de fraction de produit pur fournit des cristaux jaunes qui fondent à 156°C ; Rendement : 76 %.

En procédant selon les EXEMPLES 1 et 2 ci-dessus, on prépare les EXEMPLES 3 à 17 décrits dans le TABLEAU II ci-après.

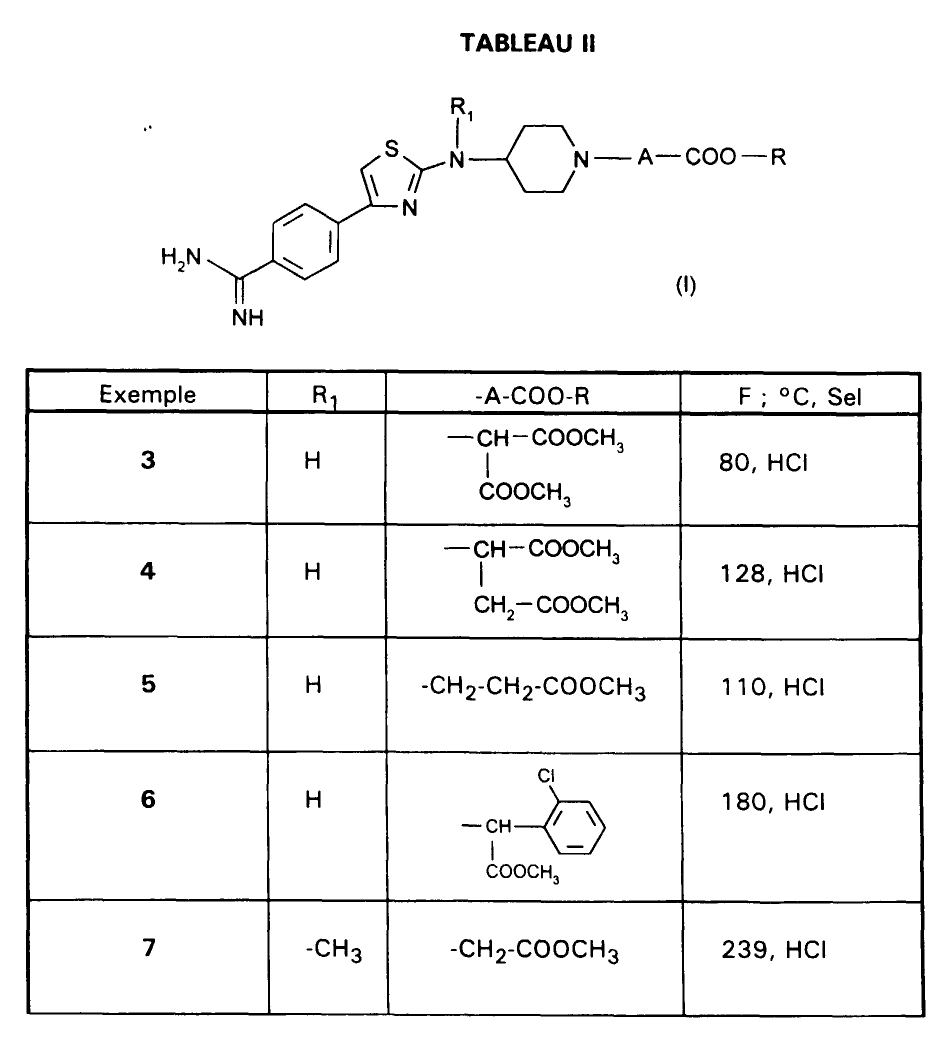

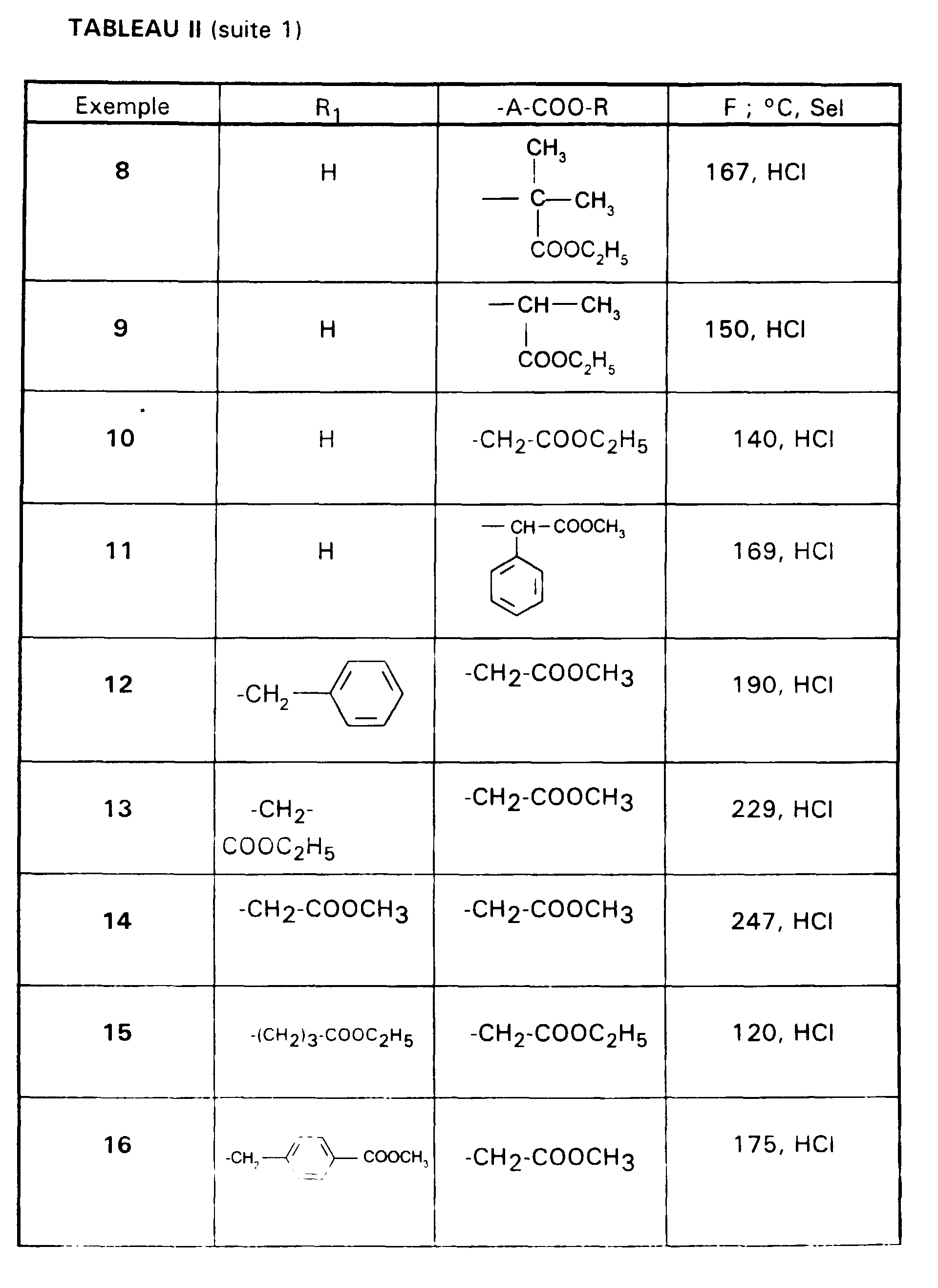

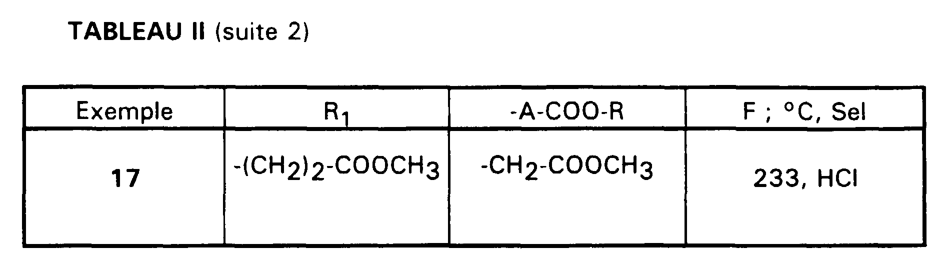

### EXEMPLE 18

### Trichlorhydrate de l'acide (4-{4-[4-(aminoiminométhyl)-phényl]-1,3-thiazol-2-yl-amino}-pipéridin-1-yl)-acétique

(I) : Y = H ; -A-COO-R = -CH₂COOH ; R₁ = H

A 20 ml d'acide chlorhydrique 6N, on ajoute 1 g de chlorhydrate de (4-{4-[4-(aminoiminométhyl)-phényl]-1,3-thiazol-2-yl-amino}-pipéridin-1-yl)-acétate de méthyle (EXEMPLE 1) et on chauffe le mélange réactionnel à reflux pendant 5 heures. On évapore à sec et on cristallise le résidu dans l'acétone pour obtenir des cristaux blancs qui fondent à 216°C ; Rendement : 96 %.

### EXEMPLE 19

### Trichlorhydrate de l'acide 3-[N-{4-[4-(aminoiminométhyl)-phényl]-1,3-thiazol-2-yl}-N-(1-carboxyméthylpipéridin-4-yl)-amino]-propionique

(I) : Y = H ; -A-COO-R = -CH₂COOH ; R₁ = -CH₂-CH₂-COOH

A 25 ml d'acide chlorhydrique 6N, on ajoute 700 mg de chlorhydrate de 3-[N-{4-[4-(aminoiminométhyl)-phényl]-1,3-thiazol-2-yl}-N-(1-éthoxycarbonylméthyl-pipéridin-4-yl)-aminol-propionate d'éthyle (EXEMPLE 2) et on chauffe le mélange réactionnel à reflux pendant 5 heures. On évapore à sec et on cristallise le résidu dans l'acétone pour obtenir des cristaux blancs qui fondent à 21 5°C ; Rendement 80 %.

En procédant selon les EXEMPLES 18 et 19 ci-dessus, on prépare les EXEMPLES 20 à 30 décrits dans le TABLEAU III ci-après.

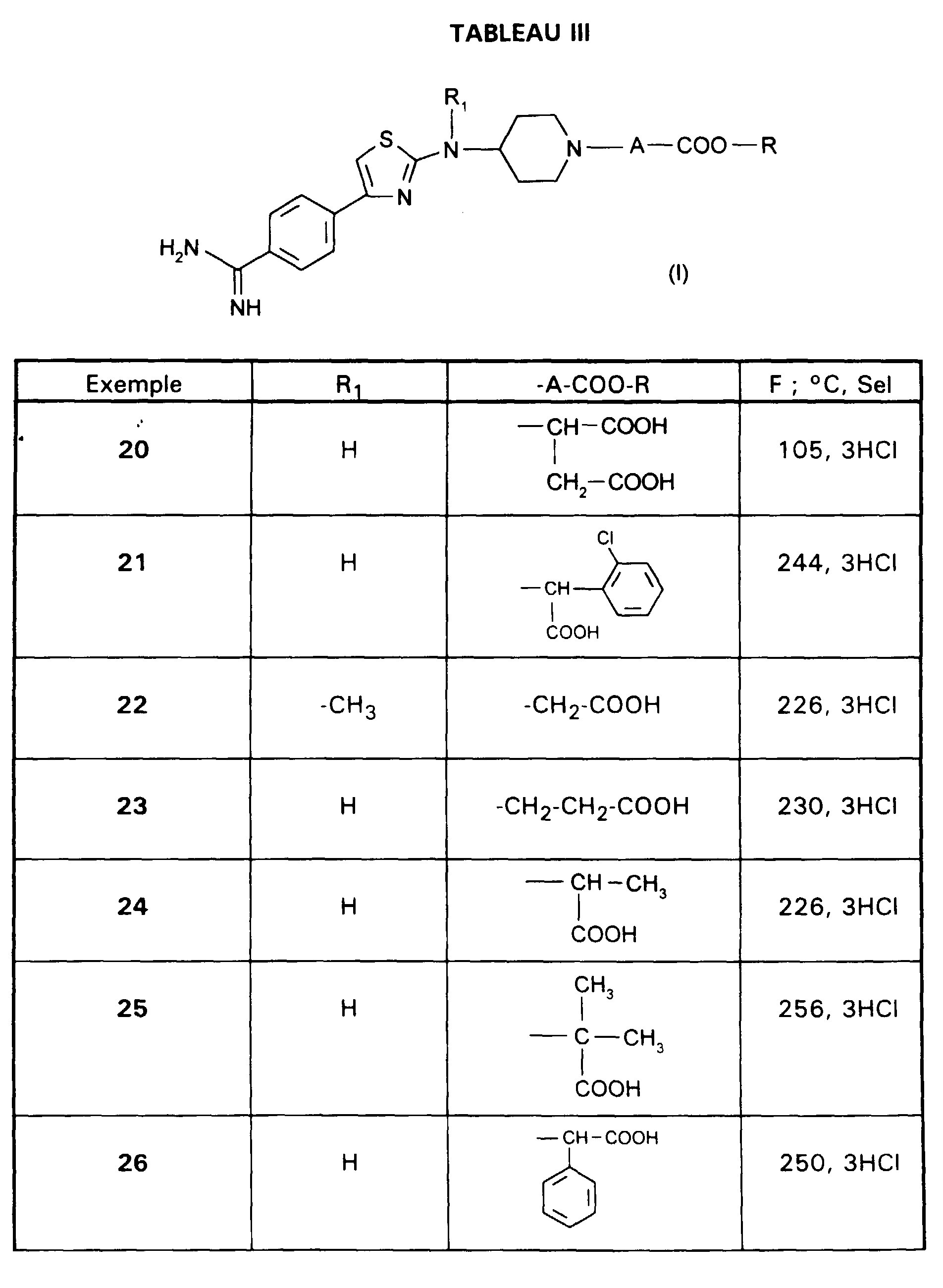

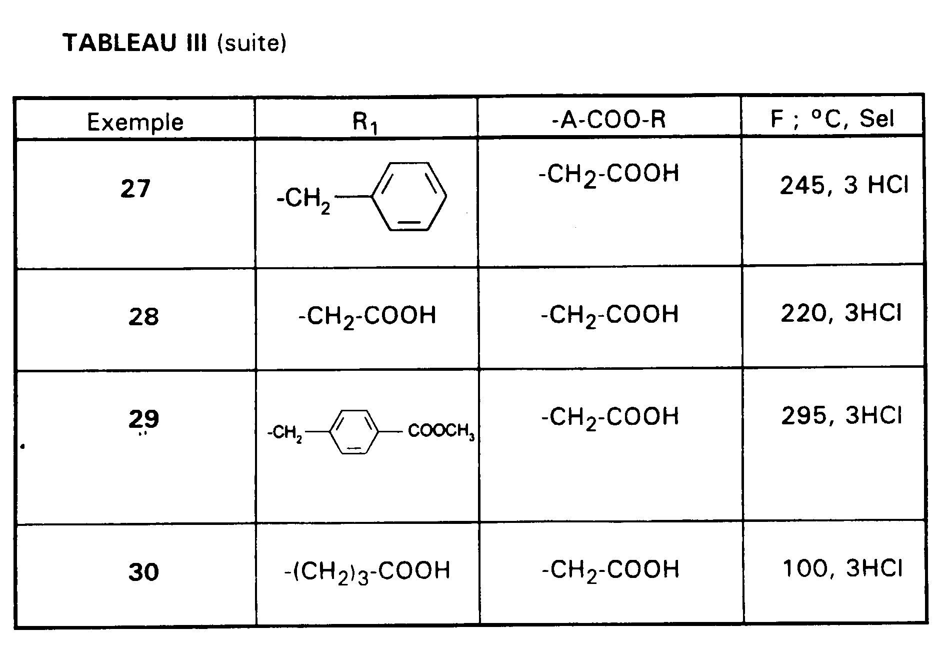

### EXEMPLE 31

### (4-{4-[4-(Aminoéthoxycarbonyliminométhyl)-phényl]-1,3-thiazol-2-yl-amino}-pipéridin-1-yl)-acétate de méthyle

### (I) : Y = -COOCH₂CH₃ ; -A-COO-R = -CH₂COOCH₃ ; R₁ = H

A 1 g de chlorhydrate de (4-{4-[4-(aminoiminométhyl)-phényl]-1,3-thiazol-2-yl-amino}-pipéridin-1-yl)-acétate de méthyle (EXEMPLE 1) dans 20 ml de N,N-diméthylformamide en présence de 0,71 ml de triéthylamine, on ajoute goutte à goutte en maintenant la température du mélange à 0°C, 0,25 ml de chloroformiate d'éthyle puis on laisse le mélange réactionnel pendant 15 minutes à cette température et on agite ensuite pendant 3 heures à température ambiante. On verse le mélange réactionnel sur de l'eau et successivement on extrait à l'acétate d'éthyle, on lave la phase organique à l'eau, on sèche sur sulfate de sodium et évapore à sec. Le résidu cristallise dans de l'éther isopropylique pour fournir des cristaux blancs qui fondent à 194°C ; Rendement 76 %.

### EXEMPLE 32

### 3-[N-{4-[4-(amino(N-éthoxycarbonyl-imino)méthyl)-phényl]-1,3-thiazol-2-yl}-N-(1-éthoxycarbonylméthylpipéridin-4-yl)-amino]-propionate d'éthyle

(I) : Y = -COOC₂H₅ ; -A-COO-R = -CH₂COOC₂H₅ ; R₁ = -CH₂-CH₂-COOC₂H₅

A 1,5 g de chlorhydrate de 3-[N-{4-[4-(aminoiminométhyl)-phényl]-1,3-thiazol-2-yl}-N-(1-éthoxycarbonylméthyl-pipéridin-4-yl)-amino]-propionate d'éthyle (EXEMPLE 2) dans 20 ml de N,N-diméthylformamide en présence de 0,84 ml de triéthylamine, on ajoute goutte à goutte en maintenant la température du mélange à 0°C, 0,29 ml de chloroformiate d'éthyle. On laisse le mélange réactionnel pendant 15 minutes à cette température puis on agite pendant 3 heures à température ambiante. On verse le mélange réactionnel sur de l'eau puis successivement on extrait à l'acétate d'éthyle, on lave la phase organique à l'eau, sèche sur sulfate de sodium et évapore à sec. Le résidu cristallise dans de l'éther isopropylique pour fournir des cristaux blancs qui fondent à 130°C ; Rendement 78 %.

En procédant selon les EXEMPLES 31 et 32 ci-dessus, on prépare les EXEMPLES 33 à 41 décrits dans le TABLEAU III ci-après par réaction des dérivés décrits dans le TABLEAU II avec les chloroformiates de formule Cl-COO-R₂ adéquats.

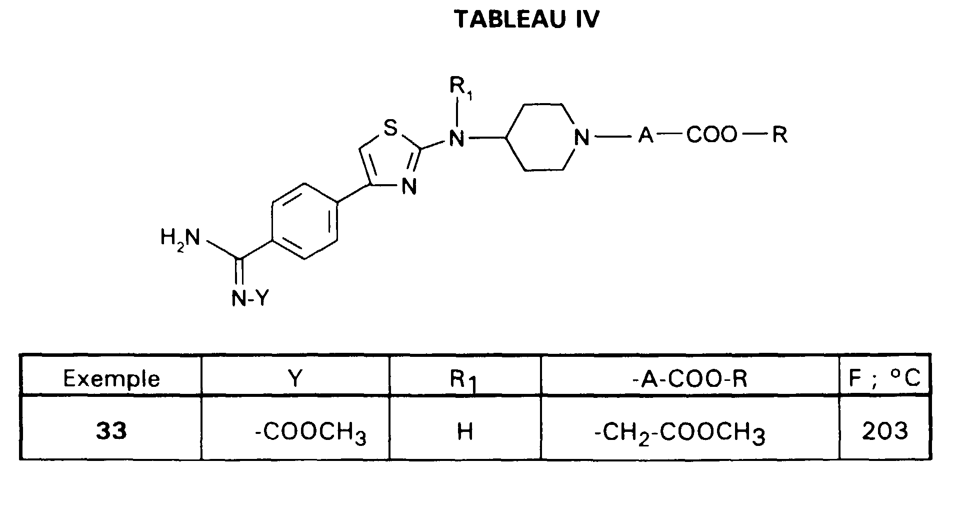

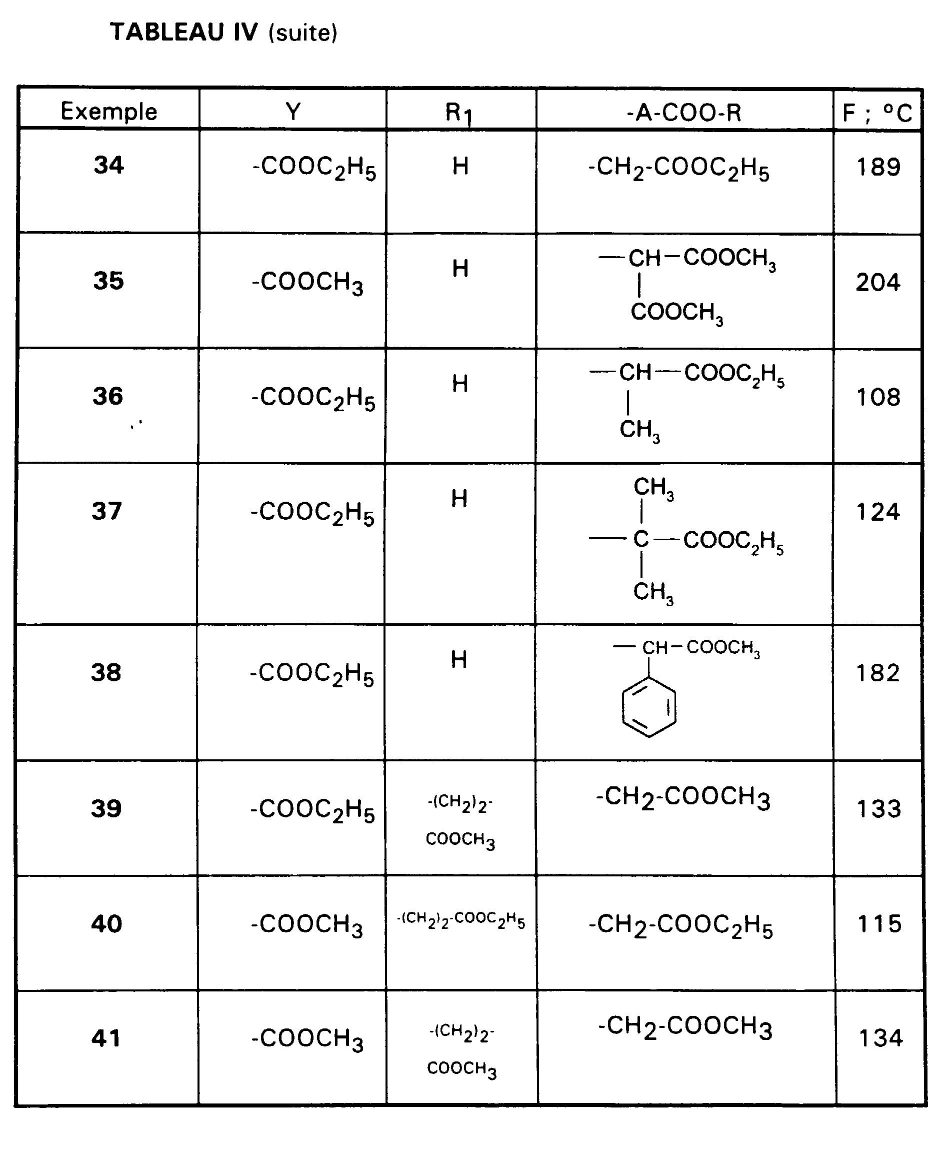

## Revendications

1. Composés de formule : 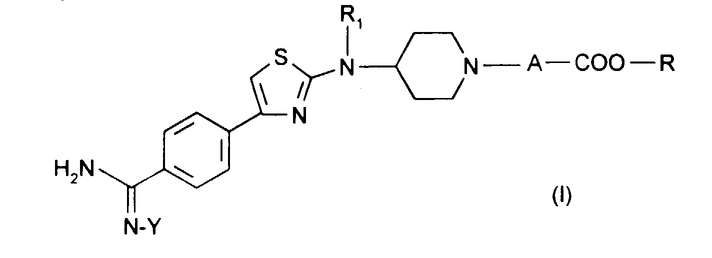 dans laquelle
- R₁ représente l'hydrogène, un alkyle en C₁-C₅, un cycloalkyle en C₃-C₈, un aralkyle dont la partie alkyle est en C₁-C₅, un groupe alcoxycarbonylalkyle ou un groupe (alcoxycarbonylaryl)alkyle dans lesquels les parties alcoxy et alkyle sont en C₁-C₃ ; un groupe carboxyalkyle ou un groupe (carboxyaryl)alkyle dans lesquels la partie alkyle est en C₁-C₃ ;
- A représente soit (i) un groupe méthylène éventuellement mono ou di-substitué par un groupe alkyle en C₁-C₅ ; par un groupe alcoxycarbonyle dont la partie alcoxy est en C₁-C₅ ; par un groupe alcoxycarbonylalkyle dans lequel les parties alcoxy et alkyle sont en C₁-C₅ ; par un groupe carboxyalkyle dont la partie alkyle est en C₁-C₅ ; par un groupe choisi parmi le phényle et le benzyle non substitués ou substitués sur le noyau aromatique par un alkyle en C₁-C₅, un alcoxy en C₁-C₅, un hydroxyle, un halogène ou un trifluorométhyle ; par un groupe pyridyle ; soit (ii) un groupe éthylène ;
- R représente l'hydrogène; un groupe alkyle en C₁-C₅ ; un groupe aryle ou un groupe aralkyle dont la partie alkyle est en C₁-C₅, lesdits groupes aryle et aralkyle étant non substitués ou substitués sur le cycle aromatique par un hydroxyle, un alcoxy en C₁-C₃, un alcanoyloxy en C₁-C₃, un halogène, un trifluorométhyle ou un alkyle en C₁-C₅ ;
- Y représente l'hydrogène ; un groupe -COOR₂ dans lequel R₂ représente un groupe alkyle en C₁-C₅, un groupe aryle ou un groupe aralkyle dont la partie alkyle est en C₁-C₅ , lesdits groupes aryle et aralkyle pouvant être éventuellement substitués sur le noyau aromatique par un alkyle en C₁-C₅ ; ou un groupe -COR₃ dans lequel R₃ représente un alkyle en C₁-C₅ ;
ou un de leurs sels,
étant entendu que aryle représente un noyau aromatique en C₆-C₁₀.

2. Composés selon la revendication 1 dans lesquels Y représente l'hydrogène, -A- représente un groupe méthylène éventuellement monosubstitué et R₁ représente l'hydrogène, un groupe méthyle, un groupe carboxyalkyle ou un groupe alcoxycarbonylalkyle, ou un de leurs sels.

3. Composés selon la revendication 1 dans lesquels Y représente un groupe -COOR₂ et -A- représente un groupe méthylène éventuellement monosubstitué dans lequel R₂ est tel que défini pour (I), ou un de leurs sels.

4. Composés selon les revendications 1 à 3 dans lesquels -A- représente un groupe -CH₂-, ou un de leurs sels.

5. Composés selon les revendications 1 à 4 dans lesquels R₁ représente l'hydrogène, un groupe carboxyalkyle ou un groupe alcoxycarbonylalkyle, R représente l'hydrogène, un groupe méthyle ou éthyle, ou un de leurs sels.

6. Composés selon les revendications 1 à 5 dans lesquels R représente un groupe méthyle ou éthyle.

7. Composés selon la revendication 1 de formule :
a) (4-{4-[4-(aminoiminométhyl)-phényl]-1,3-thiazol-2-yl-amino}-pipéridin-1-yl)- acétate de méthyle,
b) acide (4-{4-[4-(aminoiminométhyl)-phényl]-1,3-thiazol-2-yl-amino}-pipéridin-1-yl)-acétique,
c) (4-{4-[4-(amino (N-éthoxycarbonyl-imino)méthyl)-phényl]-1,3-thiazol-2-yl-amino}-pipéridin-1-yl)-acétate de méthyle,
d) (4-{4-[4-(aminoiminométhyl)-phényl]-1,3-thiazol-2-yl-amino}-pipéridin-1-yl)- acétate d'éthyle,
e) (4-{4-[4-(amino (N-éthoxycarbonyl-imino)méthyl)-phényl]-1,3-thiazol-2-yl-amino}-pipéridin-1-yl)-acétate d'éthyle,
f) acide (4-{4-[4-(aminoiminométhyl)-phényl]-1,3-thiazol-2-yl-N-carboxyméthyl-amino}-pipéridin-1-yl)-acétique,
g) 3-[N-{4-[4-(aminoiminométhyl)-phényl]-1,3-thiazol-2-yl}-N-(1-éthoxycarbonyl-méthylpipéridin-4-yl)-amino]-propionate d'éthyle,
h) acide 3-[N-{4-[4-(aminoiminométhyl)-phényl]-1,3-thiazol-2-yl}-N-(1-carboxy- méthylpipéridin-4-yl)-amino]-propionique,
i) 3-[N-{4-[4-(amino(N-éthoxycarbonyl-imino)méthyl)-phényl]-1,3-thiazol-2-yl}-N-(1-éthoxycarbonylméthylpipéridin-4-yl)-amino]-propionate d'éthyle, ou un de leurs sels.

8. Composé selon l'une des revendications 1 à 7 sous forme de mélange racémique ou d'énantiomère ou un de leurs sels.

9. Composé selon l'une des revendications 1 à 8 dans lequel le composé de formule (I) est sous forme de chlorhydrate, bromhydrate, sulfate, acétate, hydrogénosulfate, dihydrogénophosphate, méthanesulfonate, méthylsulfate, maléate, fumarate, sulfonate, 2-naphtalènesulfonate, glycolate, gluconate, citrate, iséthionate, benzoate, salicylate, ascorbate, tartrate, succinate, lactate, glutarate, toluène sulfonate, ascorbate ou encore est un sel de base inorganique par exemple un sel de métal alcalin tel qu'un sel de sodium.

10. Procédé pour la préparation des composes de formule (I) selon la revendication 1 caractérisé en ce qu'il comprend les étapes constituant à :
(a) déprotéger la fonction amine cyclique d'un composé de formule (II) : 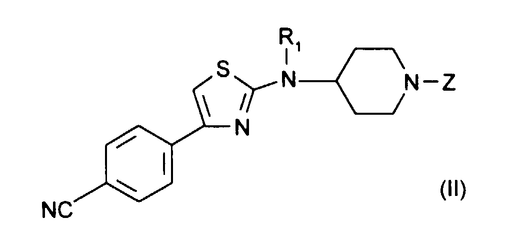 dans laquelle R₁ est tel que défini pour (I) et Z représente un groupe protecteur d'une fonction amine tel qu'un benzyle, de façon à obtenir une amine libre de formule : 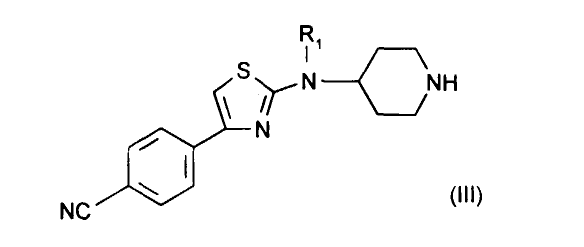 dans laquelle R₁ est tel que défini pour (I);
lorsque Z représente un benzyle, la déprotection peut être réalisée par action de chloroformiates ;
(b) N-alkyler le composé résultant de formule (III)
(i) soit par réaction avec un dérivé halogéné de formule :
X-A-COO-R (1)
dans lequel X représente un groupe nucléofuge tel qu'un groupe tosyle ou un halogène de préférence le chlore ou le brome et A et R sont tels que définis pour (I), dans un solvant choisi par exemple parmi les alcanols ou le diméthylformamide en présence d'un agent alcalin tel qu'un carbonate alcalin ou la triéthylamine ;
(ii) soit, par réaction de Michaël avec un ester α, β-insaturé de formule
CH₂ = CH-COOR (2)
ou
RCOO-CH = CH-COOR (3)
dans un alcanol où R est tel que défini pour (I),
pour obtenir un composé de formule : 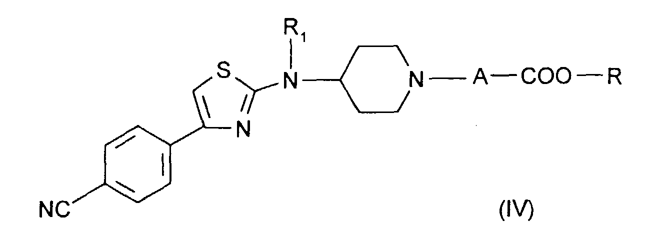 dans lequel A, R et R₁ sont tels que définis pour (I),
(c) soumettre le composé résultant à la réaction de Pinner en faisant réagir le nitrile (IV) en milieu acide dans un alcanol pour obtenir le sel imidate sur lequel on fait ensuite réagir l'ammoniac pour obtenir l'amidine de formule : 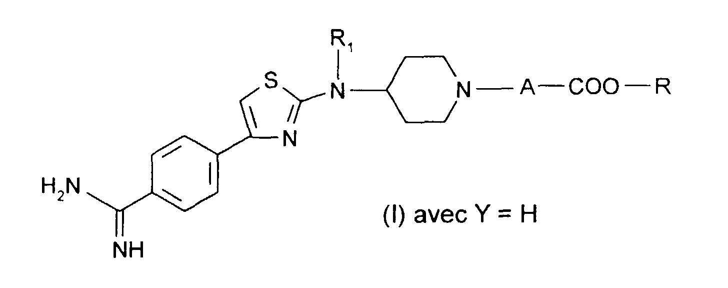 dans laquelle A, R et R₁ sont tels que définis pour (I), le composé obtenu représentant un composé de formule (I) dans laquelle Y est l'hydrogène,
(d) le cas échéant, à faire réagir l'amidine obtenue à l'étape (c)
(i) soit avec un chloroformiate de formule :
Cl-COO-R₂
dans lequel R₂ est tel que défini pour (I), dans un solvant tel que le diméthylformamide en milieu alcalin par exemple en présence de triéthylamine ou d'un carbonate alcalin pour obtenir le composé de formule: 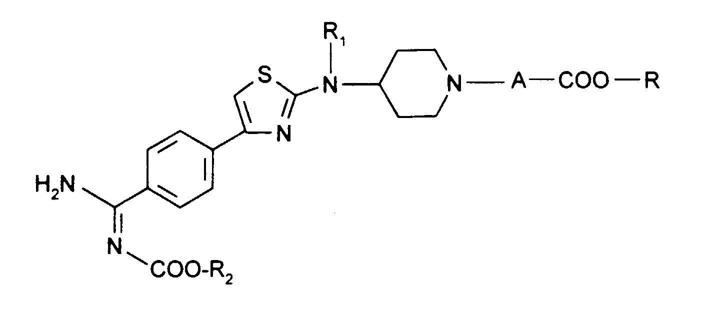 représentant un composé de formule (I) dans lequel Y représente -COO-R₂ et R₁, R₂, A et R sont tels que définis pour (I),
(ii) soit avec un agent acylant tel que Cl-CO-R₃ dans lequel R₃ est tel que défini pour (I) pour obtenir un composé de formule (I) dans lequel Y représente -CO-R₃ ou R₁, A et R sont tels que définis pour (I),
(e) le cas échéant, àn hydrolyser le précurseur ester correspondant obtenu à l'étape (c), par exemple en milieu acide en présence d'acide chlorhydrique pour obtenir un composé de formule (I) dans lequel R est hydrogène, ou un de leurs sels.

11. Composés de formule : 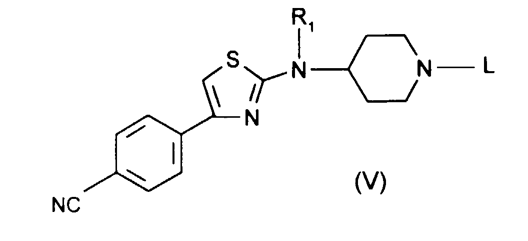 dans laquelle L représente l'hydrogène, un groupe protecteur Z tel que défini pour (II) à la revendication 10 ou un groupe -A-COOR, A, R et R₁ étant tels que définis pour (I) à la revendication 1, ou un de leurs sels.

12. Composition pharmaceutique caractérisée en ce qu'elle comprend une dose thérapeutiquement efficace d'au moins un composé selon l'une des revendications 1 à 9 sous forme de composé libre ou de sel pharmaceutiquement acceptable en association avec au moins un excipient pharmaceutique.

13. Composition pharmaceutique sous forme d'unités de dosage comprenant une dose thérapeutiquement efficace d'au moins un composé selon l'une des revendications 1 à 9 sous forme de composé libre ou de sel pharmaceutiquement acceptable, en association avec au moins un excipient pharmaceutique.

14. Composition pharmaceutique comprenant de 0,01 à 500 mg d'un composé selon l'une des revendications 1 à 9 sous forme de composé libre ou de sel pharmaceutiquement acceptable, en association avec au moins un excipient pharmaceutique.

15. Composé de formule I selon la revendication 1, dans laquelle :
- R₁ représente l'hydrogène, un alkyle C₁-C₅, un cycloalkyle en C₃-C₈, un aralkyle dont la partie alkyle est en C₁-C₅, un groupe alcoxycarbonylalkyle dans lequel les parties alcoxy et alkyle sont en C₁-C₃ ou un groupe carboxyalkyle dans lequel la partie alkyle est en C₁-C₃ ;
- A représente soit un groupe méthylène éventuellement mono ou disubstitué par un groupe alkyle en C₁-C₅ ; par un groupe alcoxycarbonyle dont la partie alcoxy est en C₁-C₅ ; par un groupe alcoxycarbonylalkyle dans lequel les parties alcoxy et alkyle sont en C₁-C₅ ; par un groupe carboxyalkyle dont la partie alkyle est en C₁-C₅ ; par un groupe phényle ou benzyle non substitué ou substitué sur le noyau aromatique par un alkyle en C₁-C₅, un alcoxy en C₁-C₅, un hydroxyle, un halogène ou un trifluorométhyle; par un groupe pyridyle ; soit un groupe éthylène ;
- R représente l'hydrogène, ou un groupe alkyle en C₁-C₅ ; un groupe aryle ou un groupe aralkyle dont la partie alkyle est en C₁-C₅, éventuellement substitué par un hydroxyle, un alcoxy en C₁-C₃, un halogène, un trifluorométhyle ou un alkyle en C₁-C₅ ;
- Y représente l'hydrogène ; un groupe -COOR₂ dans lequel R₂ représente un groupe alkyle en C₁-C₅, un groupe aryle ou un groupe aralkyle dont la partie alkyle est en C₁-C₅, éventuellement substitué par un alkyle en C₁-C₅ ; un groupe -COR₃ dans lequel R₃ représente un alkyle en C₁-C₅ ;
ou un de leurs sels.

## Patentansprüche

1. Verbindungen der Formel: 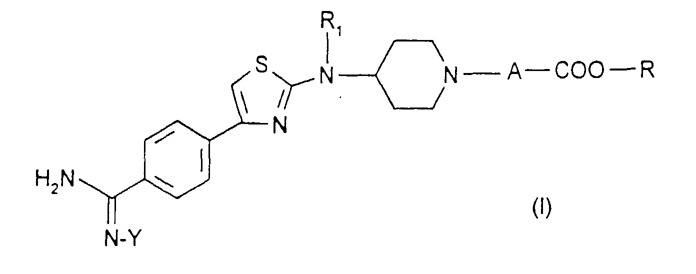 in der
- R₁ Wasserstoff, eine C₁-C₅-Alkylgruppe, eine C₃C₈-Cycloalkylgruppe, eine Aralkylgruppe, deren Alkylrest ein C₁-C₅-Rest ist, eine Alkoxycarbonylalkylgruppe oder eine (Alkoxycarbonylaryl)-alkylgruppe, worin die Alkoxy- und Alkylreste C₁-C₃-Reste sind; eine Carboxyalkylgruppe oder eine (Carboxyaryl)-alkylgruppe, in denen der Alkylrest ein C₁-C₃-Rest ist;
- A entweder (i) eine Methylengruppe, die gegebenenfalls mono- oder disubstituiert ist durch eine C₁-C₅-Alkylgruppe; durch eine Alkoxycarbonylgruppe, deren Alkoxyrest ein C₁-C₅-Rest ist; durch eine Alkoxycarbonylalkylgruppe, in der die Alkoxy- und Alkylreste C₁-C₅-Reste sind; durch eine Carboxyalkylgruppe, deren Alkylrest ein C₁-C₅-Rest ist; durch eine Gruppe ausgewählt aus Phenyl und Benzyl, die unsubstituiert oder am aromatischen Kern durch eine C₁-C₅-Alkylgruppe, eine C₁-C₅-Alkoxygruppe, eine Hydroxylgruppe, ein Halogenatom oder eine Trifluormethylgruppe substituiert sind; oder durch eine Pyridylgruppe; oder (ii) eine Ethylengruppe;
- R Wasserstoff; eine C₁-C₅-Alkylgruppe; eine Arylgruppe oder eine Aralkylgruppe, deren Alkylrest ein C₁-C₅-Rest ist, welche Arylgruppen und Aralkylgruppen unsubstituiert sind oder am aromatischen Kern durch eine Hydroxylgruppe, eine C₁-C₃-Alkoxygruppe, eine C₁-C₃-Alkanoyloxygruppe, ein Halogenatom, eine Trifluormethylgruppe oder eine C₁-C₅-Alkylgruppe substituiert sind; und
- Y Wasserstoff; eine Gruppe -COOR₂, worin R₂ eine C₁-C₅-Alkylgruppe, eine Arylgruppe oder eine Aralkylgruppe, deren Alkylrest ein C₁-C₅-Rest ist, wobei die Arylgruppen und Aralkylgruppen gegebenenfalls am aromatischen Kern durch eine C₁-C₅-Alkylgruppe substituiert sind, darstellt; oder eine Gruppe -COR₃, worin R₃ eine C₁-C₅-Alkylgruppe darstellt; bedeuten
oder eines ihrer Salze,
wobei es sich versteht, daß Aryl für einen aromatischen C₆-C₁₀-Kern steht.

2. Verbindungen nach Anspruch 1, worin Y Waserstoff, -A- eine gegebenenfalls monosubstituierte Methylengruppe und R₁ Wasserstoff, eine Methylgruppe, eine Carboxyalkylgruppe oder eine Alkoxycarbonylalkylgruppe bedeuten, oder eines ihrer Salze.

3. Verbindungen nach Anspruch 1, worin Y eine Gruppe -COOR₂ und -Reine gegebenenfalls monosubstituierte Methylengruppe bedeuten, worin R₂ die bezüglich der Formel (I) angegebenen Bedeutungen besitzt, oder eines ihrer Salze.

4. Verbindungen nach den Ansprüchen 1 bis 3, worin -A- eine Gruppe -CH₂- bedeutet, oder eines ihrer Salze.

5. Verbindungen nach den Ansprüchen 1 bis 4, worin R₁ Wasserstoff, eine Carboxyalkylgruppe oder eine Alkoxycarbonylalkylgruppe und R Wasserstoff, eine Methylgruppe oder eine Ethylgruppe bedeuten, oder eines ihrer Salze.

6. Verbindungen nach den Ansprüchen 1 bis 5, worin R eine Methylgruppe oder eine Ethylgruppe darstellt.

7. Verbindungen nach Anspruch 1 der Formel:
a) (4-{4-[4-(Aminoiminomethyl)-phenyl]-1,3-thiazol-2-yl-amino}-piperidin-1-yl)-essigsäuremethylester,
b) (4-{4-[4-(Aminoiminomethyl)-phenyl]-1,3-thiazol-2-yl-amino}-piperidin-1-yl)-essigsäure,
c) (4-{4-[4-(Amino-(N-ethoxycarbonyl-imino)-methyl)-phenyl]-1,3-thiazol-2-yl-amino}-piperidin-1-yl)-essigsäuremethylester,
d) (4-{4-[4-(Aminoiminomethyl)-phenyl]-1,3-thiazol-2-yl-amino}-piperidin-1-yl)-essigsäureethylester,
e) (4-{4-[4-(Amino-(N-ethoxycarbonyl-imino)-methyl)-phenyl]-1,3-thiazol-2-yl-amino}-piperidin-1-yl)-essigsäureethylester,
f) (4-{4-[4-(Aminoiminomethyl)-phenyl]-1,3-thiazol-2-yl-N-carboxymethyl-amino}-piperidin-1-yl)-essigsäure,
g) 3-[N-{4-[4-(Aminoiminomethyl)-phenyl]-1,3-thiazol-2-yl}-N-(1-ethoxycarbonyl-methylpiperidin-4-yl)-amino]-propionsäureethylester,
h) 3-[N-{4-[4-[Aminoiminomethyl)-phenyl]-1,3-thiazol-2-yl}-N-(1-carboxy-methylpiperidin-4-yl]-amino]-propionsäure,
i) 3-[N-{4-[4-(Amino-(N-ethoxycarbonyl-imino)-methyl)-phenyl]-1,3-thiazol-2-yl}-N-(1-ethoxycarbonyl-methylpiperidin-4-yl)-amino]-propionsäureethylester, oder eines ihrer Salze.

8. Verbindung nach einem der Ansprüche 1 bis 7 in Form einer racemischen Mischung oder des Enantiomeren oder eines ihrer Salze.

9. Verbindung nach einem der Ansprüche 1 bis 8, worin die Verbindung der Formel (I) in Form des Hydrochlorids, Hydrobromids, Sulfats, Acetats, Hydrogensulfats, Dihydrogenphosphats, Methansulfonats, Methylsulfats, Maleats, Fumarats, Sulfonats, 2-Naphthalinsulfonats, Glykolats, Gluconats, Citrats, Isethionats, Benzoats, Salicylats, Ascorbats, Tartrats, Succinats, Lactats, Glutarats, Toluolsulfonats oder Ascorbats vorliegt oder ein Salz einer anorganischen Base ist, beispielsweise ein Salze eines Alkalimetalls, wie ein Natriumsalz.

10. Verfahren zur Herstellung der Verbindungen der Formel (I) nach Anspruch 1, dadurch gekennzeichnet, daß es die folgenden Stufen umfaßt, welche darin bestehen:
(a) die Schutzgruppe der cyclischen Aminfunktion einer Verbindung der Formel (II) 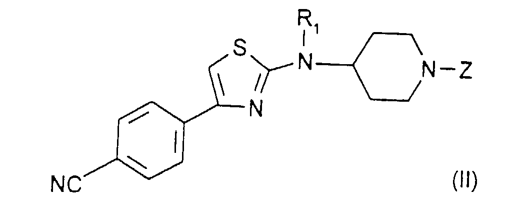 in der R₁ die bezüglich (I) angegebenen Bedeutungen besitzt und Z eine Schutzgruppe für eine Aminfunktion, wie eine Benzylgruppe darstellt, abzuspalten unter Erhalt eines freien Amins der Formel: 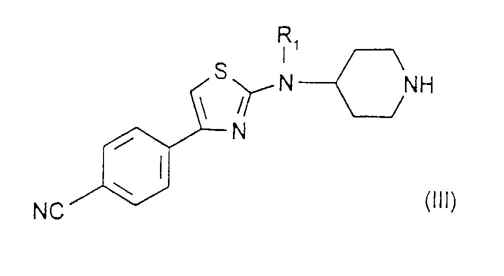 in der R₁ die bezüglich (I) angegebenen Bedeutungen besitzt;
wobei, denn Z eine Benzylgruppe darstellt, die Abspaltung der Schutzgruppe durch Einwirkung von Chlorameisensäureestern erreicht werden kann;
(b) N-Alkylierung der gebildeten Verbindung der Formel (III)
(i) entweder durch Umsetzung mit einem Halogenderivat der Formel:
X-A-COO-R (1)
in der X eine nucleofuge Gruppe, wie eine Tosylgruppe oder ein Halogenatom, vorzugsweise ein Chlor- oder Bromatom darstellt, und A und R die bezüglich (I) angegebenen Bedeutungen besitzen, in einem Lösungsmittel ausgewählt aus beispielsweise Alkanolen oder Dimethylformamid, in Gegenwart eines alkalischen Mittels, wie eines Alkalimetallcarbonats oder von Triethylamin;
(ii) oder durch Michael-Reaktion mit einem α,β-ungesättigten Ester der Formel
CH₂ = CH - COOR (2) (2)
oder
RCOO - CH = CH - COOR (3)
worin R die bezüglich (I) angegebenen Bedeutungen besitzt, in einem Alkanol zur Bildung einer Verbindung der Formel: 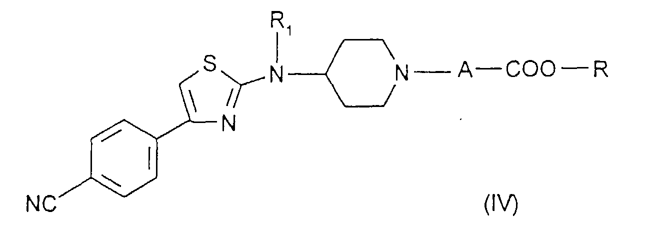 in der A, R und R₁ die bezüglich (I) angegebenen Bedeutungen besitzen,
(c) Umsetzen der gebildeten Verbindung nach der Pinner-Reaktion durch Umsetzen des Nitrils (IV) in saurem Medium in einem Alkanol zur Bildung des Imidatsalzes, welches man anschließend
mit Ammoniak umsetzt zur Bildung des Amidins der Formel: 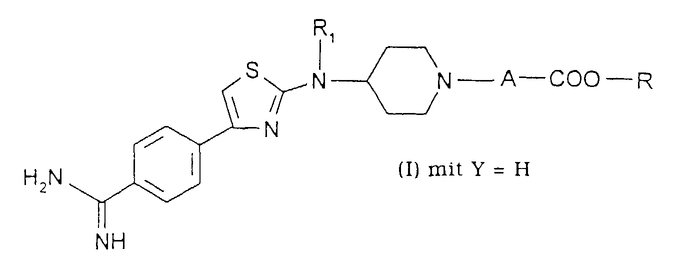 in der A, R und R₁ dic bezüglich (I) angegebenen Bedeutungen besitzt, wobei die erhaltene Verbindung eine Verbindung der Formel (I) darstellt, in der Y Wasserstoff bedeutet,
(d) gegebenenfalls Umsetzen des in der Stufe (c) erhaltenen Amidins
(i) entweder mit einem Chlorameisensäureester der Formel:
Cl- COO - R₂
in der R₂ die bezüglich (I) angegebenen Bedeutungen besitzt, in einem Lösungsmittel, wie Dimethylformamid, in alkalischem Medium, beispielsweise in Gegenwart von Triethylamin oder eines Alkalimetallcarbonats, zur Bildung der Verbindung der Formel: 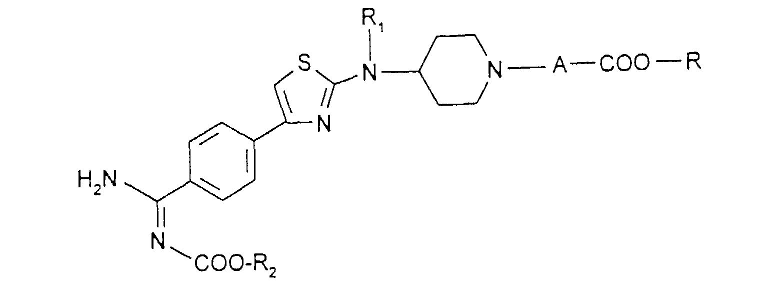 welche eine Verbindung der Formel (I) darstellt, in der Y -COO-R₂ darstellt und R₁, R₂, A und R die bezüglich (I) angegebenen Bedeutungen besitzen,
(ii) oder mit einem Acylierungsmittel, wie Cl-CO-R₃, worin R₃ die bezüglich (I) angegebenen Bedeutungen besitzt, zur Bildung einer Verbindung der Formel (I), in der Y-CO-R₃ darstellt und R₁, A und R die bezüglich (I) angegebenen Bedeutungen besitzen,
(e) gegebenenfalls Hydrolyse des in der Stufe (c) erhaltenen entsprechenden Vorläufer-Esters, beispielsweise in saurem Medium in Gegenwart von Chlorwasserstoffsäure, zur Bildung einer Verbindung der Formel (I), in der R Wasserstoff bedeutet, oder eines ihrer Salze.

11. Verbindungen der Formel: 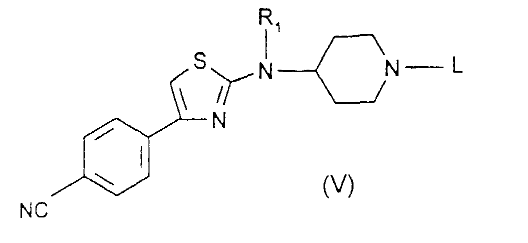 in der L Wasserstoff, eine Schutzgruppe Z, wie sie in Anspruch 10 bezüglich (II) definiert worden ist, oder eine Gruppe -A-COOR bedeutet, während A, R und R₁ die in Anspruch 1 bezüglich (I) angegebenen Bedeutungen besitzen, oder eines ihrer Salze.

12. Pharmazeutische Zubereitung, dadurch gekennzeichnet, daß sie eine therapeutisch wirksame Dosis mindestens einer Verbindung nach einem der Ansprüche 1 bis 9 in Form der freien Verbindung oder des pharmazeutisch annehmbaren Salzes in Kombination mit mindestens einem pharmazeutischen Trägermaterial umfaßt.

13. Pharmazeutische Zubereitung in Form von Dosiseinheitsformen enthaltend eine therapeutisch wirksame Dosis mindestens einer Verbindung nach einem der Ansprüche 1 bis 9 in Form der freien Verbindung oder des pharmazeutisch annehmbaren Salzes in Kombination mit mindestens einem pharmazeutischen Trägermaterial.

14. Pharmazeutische Zubereitung enthaltend 0,01 bis 500 mg einer Verbindung nach einem der Ansprüche 1 bis 9 in Form der freien Verbindung oder des pharmazeutisch annehmbaren Salzes, in Kombination mit einem oder mehreren pharmazeutischen Trägermaterialien.

15. Verbindung der Formel I nach Anspruch 1, worin:
- R₁ Wasserstoff, eine C₁-C₅-Alkylgruppe, eine C₃-C₈-Cycloalkylgruppe, eine Aralkylgruppe, deren Alkylrest ein C₁-C₅-Rest ist, eine Alkoxycarbonylalkylgruppe, deren Alkoxy- und Alkylreste C₁-C₃-Reste sind oder eine Carboxyalkylgruppe, in der der Alkylrest ein C₁-C₃-Rest ist;
- A entweder eine Methylengruppe, die gegebenenfalls mono- oder disubstituiert ist mit einer C₁-C₅-Alkylgruppe; mit einer Alkoxycarbonylgruppe, deren Alkoxyrest ein C₁-C₅-Rest ist; durch eine Alkoxycarbonylalkylgruppe, in der die Alkoxy- und Alkylreste C₁-C₅-Reste sind; durch eine Carboxyalkylgruppe, deren Alkylrest ein C₁-C₅-Rest ist; durch eine Phenylgruppe oder eine Benzygruppe, die unsubstituiert sind oder am aromatischen Kern durch eine C₁-C₅-Alkylgruppe, eine C₁-C₅-Alkoxygruppe, eine Hydroxygruppe, ein Halogenatom oder eine Trifluormethylgruppe substituiert sind; durch eine Pyridylgruppe; oder eine Ethylengruppe;
- R Wasserstoff oder eine C₁-C₅-Alkylgruppe; eine Arylgruppe oder eine Aralkylgruppe, deren Alkylrest ein C₁-C₅-Rest ist, der gegebenenfalls durch eine Hydroxylgruppe, eine C₁-C₃-Alkoxygruppe, ein Halogenatom, eine Trifluormethylgruppe oder eine C₁-C₅-Alkylgruppe substituiert ist; und
- Y Wasserstoff; eine Gruppe -COOR₂, in der R₂ eine C₁-C₅-Alkylgruppe, eine Arylgruppe oder eine Aralkylgruppe, deren Alkylrest ein C₁-C₅-Rest ist, vier gegebenenfalls durch eine C₁-C₅-Alkylgruppe substituiert ist, darstellt; eine Gruppe -COR₃, in der R₃ eine C₁-C₅-Alkylgruppe darstellt, bedeuten; oder eines ihrer Salze.

## Claims

1. Compounds of formula: 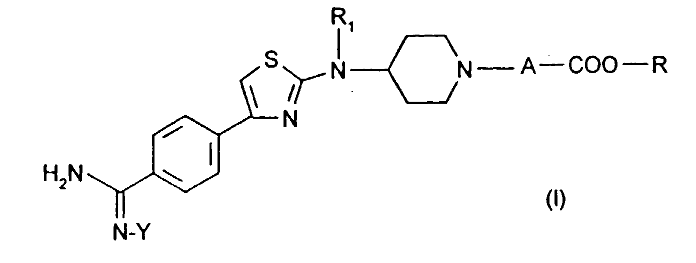 wherein
- R₁ denotes hydrogen, a C₁₋₅-alkyl, a C₃₋₈-cycloalkyl, an aralkyl the alkyl moiety of which is C₁₋₅, an alkoxycarbonylalkyl group or an (alkoxycarbonylaryl)alkyl group wherein the alkoxy and alkyl moieties are C₁-C₃; a carboxyalkyl group or a (carboxyaryl)alkyl group wherein the alkyl moiety is C₁-C₃;
- A denotes either (i) a methylene group optionally mono- or disubstituted by a C₁₋₅-alkyl group; by an alkoxycarbonyl group the alkoxy moiety of which is C₁₋₅; by an alkoxycarbonylalkyl group wherein the alkoxy and alkyl moieties are C₁₋₅; by a carboxyalkyl group the alkyl moiety of which is C₁₋₅; by a group selected from among phenyl and benzyl which may be unsubstituted or substituted at the aromatic nucleus by a C₁₋₅-alkyl, C₁₋₅-alkoxy, a hydroxyl, a halogen or a trifluoromethyl; by a pyridyl group; or (ii) an ethylene group;
- R denotes hydrogen; a C₁₋₅-alkyl group; an aryl group or an aralkyl group the alkyl moiety of which is C₁₋₅, said aryl and aralkyl groups being unsubstituted or substituted on the aromatic ring by a hydroxyl, a C₁₋₃-alkoxy, a C₁₋₃-alkanoyloxy, a halogen, a trifluoromethyl or a C₁₋₅-alkyl;
- Y denotes hydrogen; a -COOR₂ group wherein R₂ denotes a C₁₋₅-alkyl group, an aryl group or an aralkyl group the alkyl moiety of which is C₁₋₅, said aryl and aralkyl groups optionally being substituted at the aromatic nucleus by a C₁₋₅-alkyl; or a group -COR₃ wherein R₃ denotes a C₁₋₅-alkyl;
or one of the salts thereof,
it being understood that aryl denotes a C₆₋₁₀-aromatic nucleus.

2. Compounds according to claim 1 wherein Y denotes hydrogen, -A- denotes an optionally monosubstituted methylene group and R₁ denotes hydrogen, a methyl group, a carboxyalkyl group or an alkoxycarbonylalkyl group, or one of the salts thereof.

3. Compounds according to claim 1 wherein Y denotes a -COOR₂ group and -A- denotes an optionally monosubstituted methylene group wherein R₂ is as defined for (I), or one of the salts thereof.

4. Compounds according to claims 1 to 3 wherein -A- denotes a group -CH₂-, or one of the salts thereof.

5. Compounds according to claims 1 to 4 wherein R₁ denotes hydrogen, a carboxylalkyl group or an alkoxycarbonylalkyl group, R denotes hydrogen or a methyl or ethyl group, or one of the salts thereof.

6. Compounds according to claims 1 to 5 wherein R denotes a methyl or ethyl group.

7. Compounds according to claim 1 of formula:
a) methyl (4-{4-[4-(aminoiminomethyl)-phenyl]-1,3-thiazol-2-yl-amino}-piperidin-1-yl)-acetate,
b) (4-{4-[4-(aminoiminomethyl)-phenyl]-1,3-thiazol-2-yl-amino}-piperidin-1-yl)-acetic acid,
c) methyl (4-{4-[4-(amino-(N-ethoxycarbonyl-imino)methyl)-phenyl]-1,3-thiazol-2-yl-amino}-piperidin-1-yl)-acetate,
d) ethyl (4-{4-[4-(aminoiminomethyl)-phenyl]-1,3-thiazol-2-yl-amino}-piperidin-1-yl)-acetate,
e) ethyl (4-{4-[4-(amino-(N-ethoxycarbonyl-imino)methyl)-phenyl]-1,3-thiazol-2-yl-amino}-piperidin-1-yl)-acetate
f) (4-{4-[4-(aminoiminomethyl)-phenyl]-1,3-thiazol-2-yl-N-carboxymethyl-amino}-piperidin-1-yl)-acetic acid,
g) ethyl 3-[N-{4-[4-(aminoiminomethyl)-phenyl]-1,3-thiazol-2-yl}-N-(1-ethoxycarbonyl-methylpiperidin-4-yl)-amino]-propionate,
h) 3-[N-{4-[4-(aminoiminomethyl)-phenyl]-1,3-thiazol-2-yl}-N-(1-carboxy-methylpiperidin-4-yl)-amino]-propionic acid,
i) ethyl 3-[N-{4-[4-(amino(N-ethoxycarbonyl-imino)methyl)-phenyl]-1,3-thiazol-2-yl}-N-(1-ethoxycarbonylmethylpiperidin-4-yl)-amino]-propionate, or one of the salts thereof.

8. Compound according to one of claims 1 to 7 in the form of a racemic mixture or a mixture of enantiomers or one of the salts thereof.

9. Compound according to one of claims 1 to 8 wherein the compound of formula (I) is in the form of a hydrochloride, hydrobromide, sulphate, acetate, hydrogen sulphate, dihydrogen phosphate, methanesulphonate, methyl sulphate, maleate, fumarate, sulphonate, 2-naphthalenesulphonate, glycolate, gluconate, citrate, isothionate, benzoate, salicylate, ascorbate, tartrate, succinate, lactate, glutarate, toluene sulphonate or ascorbate or is a salt of an inorganic base, for example an alkali metal salt such as a sodium salt.

10. Process for preparing compounds of formula (I) according to claim 1, characterised in that it comprises steps which consist in:
(a) deprotecting the cyclic amine function of a compound of formula (II) 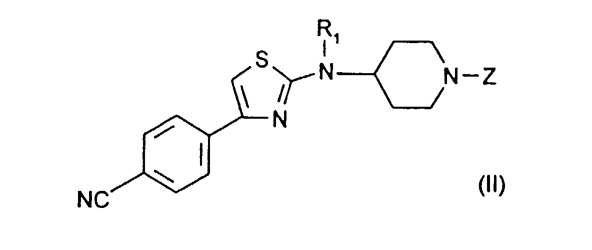 wherein R₁ is as defined for (I) and Z denotes a protecting group for an amine function such as a benzyl, so as to obtain a free amine of formula: 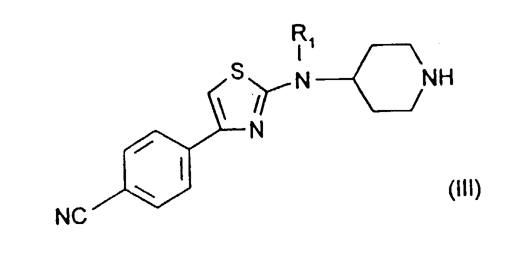 wherein R₁ is as defined for (I);
when Z denotes a benzyl, the deprotection may be carried out by the action of chloroformates;
(b) N-alkylating the resulting compound of formula (III)
(i) either by reacting with a halo derivative of formula:
X-A-COO-R (1)
wherein X denotes a nucleofugic group such as a tosyl group or a halogen, preferably chlorine or bromine, and A and R are as defined for (I), in a solvent selected, for example, from among the alkanols or dimethylformamide in the presence of an alkaline agent such as an alkali metal carbonate or triethylamine;
(ii) or by Michael reaction with an α,β-unsaturated ester of formula
CH₂ = CH-COOR (2)
or
RCOO-CH = CH-COOR (3)
in an alkanol wherein R is as defined for (I),
in order to obtain a compound of formula: 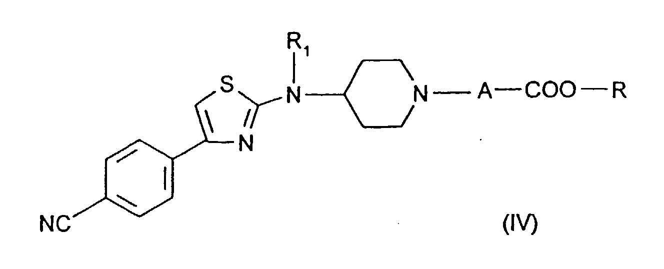 wherein A, R and R₁ are as defined for (I),
(c) subjecting the resulting compound to a Pinner reaction by reacting the nitrile (IV) in an acid medium in an alkanol in order to obtain the imidate salt which is subsequently reacted with ammonia in order to obtain the amidine of formula: 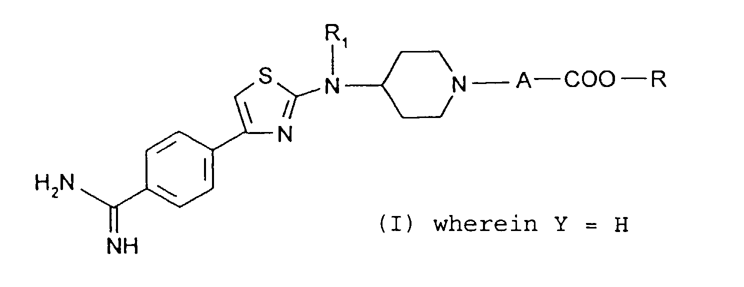 wherein A, R and R₁ are as defined for (I), the compound obtained denoting a compound of formula (I) wherein Y is hydrogen,
(d) if necessary, reacting the amidine obtained in step (c)
(i) either with a chloroformate of formula:
Cl-COO-R₂
wherein R₂ is as defined for (I), in a solvent such as dimethylformamide in an alkaline medium, for example in the presence of triethylamine or an alkali metal carbonate, to obtain the compound of formula: 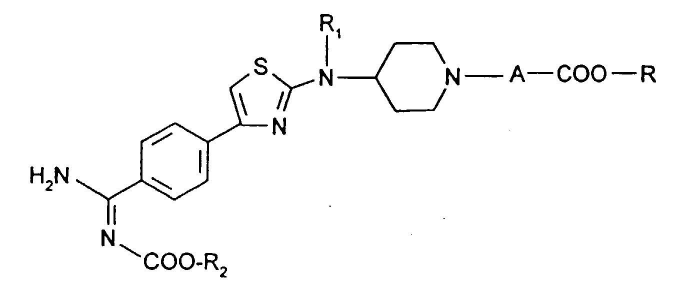 representing a compound of formula (I) wherein Y denotes -COO-R₂ and R₁, R₂, A and R are as defined for (I),
(ii) or with an acylating agent such as Cl-CO-R₃ wherein R₃ is as defined for (I) in order to obtain a compound of formula (I) wherein Y denotes -CO-R₃ or R₁, A and R are as defined for (I),
(e) if necessary, hydrolysing the corresponding ester precursor obtained in step (c), for example in an acid medium in the presence of hydrochloric acid, to obtain a compound of formula (I) wherein R is hydrogen, or one of the salts thereof.

11. Compounds of formula: 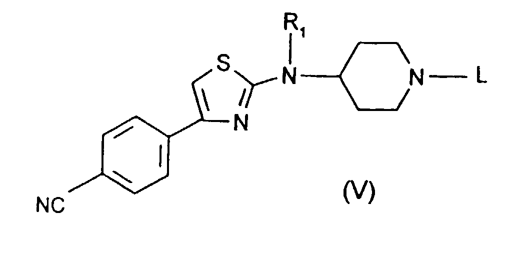 wherein L denotes hydrogen, a protecting group Z as defined for (II) in claim 10 or a group -A-COOR, wherein A, R and R₁ are as defined for (I) in claim 1, or one of the salts thereof.

12. Pharmaceutical composition, characterised in that it contains a therapeutically effective dose of at least one compound according to one of claims 1 to 9 in the form of a free compound or pharmaceutically acceptable salt combined with at least one pharmaceutical excipient.

13. Pharmaceutical composition in the form of dosage units containing a therapeutically effective dose of at least one compound according to one of claims 1 to 9 in the form of a free compound or pharmaceutically acceptable salt, combined with at least one pharmaceutical excipient.

14. Pharmaceutical composition containing 0.01 to 500 mg of a compound according to one of claims 1 to 9 in the form of a free compound or pharmaceutically acceptable salt, combined with at least one pharmaceutical excipient.

15. Compound of formula I according to claim 1,
wherein:
- R₁ denotes hydrogen, a C₁₋₅-alkyl, a C₃₋₈-cycloalkyl, an aralkyl wherein the alkyl moiety is C₁₋₅, an alkoxycarbonylalkyl group wherein the alkoxy and alkyl alkyl moieties are C₁-C₃ or a carboxyalkyl group wherein the alkyl moiety is C₁-C₃;
- A denotes either a methylene group optionally mono- or disubstituted by a C₁₋₅-alkyl group; by an alkoxycarbonyl group wherein the alkoxy moiety is C₁₋₅; by an alkoxycarbonylalkyl group wherein the alkoxy and alkyl moieties are C₁₋₅; by a carboxyalkyl group wherein the alkyl moiety is C₁₋₅; by a phenyl or benzyl group which is unsubstituted or substituted at the aromatic nucleus by a C₁₋₅-alkyl, a C₁₋₅-alkoxy, a hydroxyl, a halogen or a trifluoromethyl; by a pyridyl group; or an ethylene group;
- R denotes hydrogen or a C₁₋₅-alkyl group; an aryl group or an aralkyl group wherein the alkyl moiety is C₁₋₅, optionally substituted by a hydroxyl, a C₁₋₃-alkoxy, a halogen, a trifluoromethyl or a C₁₋₅-alkyl;
- Y denotes hydrogen; a -COOR₂ group wherein R₂ denotes a C₁₋₅-alkyl group, an aryl group or an aralkyl group wherein the alkyl moiety is C₁₋₅, optionally substituted by a C₁₋₅-alkyl; a -COR₃ group wherein R₃ denotes a C₁₋₅-alkyl;
or one of the salts thereof.
